# EUROPEAN PATENT APPLICATION

(11) **EP 4 635 982 A1**
(43) Date of publication of application: **22.10.2025**
(21) Application number: 23905875.3
(22) Date of filing: 18.12.2023
(51) Int. Cl.: C07K 16/28, C07K 16/46, A61K 39/395, A61P 35/00, A61P 37/06, A61P 37/02, A61P 31/00, G01N 33/577, G01N 33/68

(54) **NKP46 ANTIBODY AND USE THEREOF**

(30) Priority: 20.12.2022 CN 202211644532
(71) Applicant: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Latscha Schöllhorn Partner AG
(86) International application number: PCT/CN2023/139438
(87) International publication number: WO 2024/131711

(57) **Abstract**

Provided is an antibody or antigen-binding fragment. The antibody or antigen-binding fragment includes a CDR selected from at least one of: heavy chain variable region CDRs with amino acid sequences as set forth in SEQ ID NO: 1 to SEQ ID NO: 3, SEQ ID NO: 7 to SEQ ID NO: 9, or SEQ ID NO: 13 to SEQ ID NO: 15, or conservative modification forms thereof; or light chain variable region CDRs with amino acid sequences as set forth in SEQ ID NO: 4 to SEQ ID NO: 6, SEQ ID NO: 10 to SEQ ID NO: 12, or SEQ ID NO: 16 to SEQ ID NO: 18, or conservative modification forms thereof.

## Description

### FIELD

The present disclosure belongs to the technical field of biopharmaceutical. Specifically, the present disclosure relates to an NKp46 antibody and uses thereof. More specifically, the present disclosure relates to an antibody or antigen-binding fragment, a recombinant protein, a multispecific antibody, a nucleic acid molecule, an expression vector, a recombinant cell, a pharmaceutical composition, a conjugate, a kit, and use thereof.

### BACKGROUND

NK cell is a very important innate lymphocyte in the body, and plays an important role in anti-virus and anti-tumor. NK cells are also highly associated with the occurrence and development of autoimmune diseases, transplant rejection, aseptic inflammation, and excessive inflammation induced by pathogenic microorganism infections.

NK cells express a variety of receptors on their surface, including activating receptors such as NKp46, NKp30, and NKG2D, as well as inhibitory receptors such as NKG2A, KIR2DL3, TIGIT, and PVRIG. Ligands of activating receptors, such as B7H6 and MICA/B, are upregulated in cases of cellular malignant transformation (tumor), autoimmune inflammation, or infectious inflammation. NK cells recognize and bind to corresponding ligands through activating receptors encoded by germline genes, thereby recognizing and killing tumor cells.

NKp46 is an important NK cell activating receptor. It transmits an activation signal to promote the NK cell to kill the tumor after binding to the ligand on the surface of tumor cells. Currently, the only publicly disclosed patents for NKp46 antibodies worldwide are US20190367609A1 and US10716864. Validation has shown that the NKp46 antibody disclosed in US10716864 does not bind to monkey NKp46, which is unfavorable for the safety evaluation of drugs in non-human primates.

Therefore, it is urgently needed to develop an antibody that can bind to human NKp46 and monkey NKp46 as well as promote NK cell activation.

### SUMMARY

The present disclosure aims to solve at least one of the technical problems existing in the related art to a certain extent. To this end, the present disclosure provides an NKp46 antibody or antigen-binding fragment, which can bind to NKp46 and promote activation of NK cells.

In a first aspect of the present disclosure, the present disclosure provides an antibody or antigen-binding fragment. According to an embodiment of the present disclosure, the antibody or antigen-binding fragment includes a CDR selected from at least one of: heavy chain variable region CDRs with amino acid sequences as set forth in SEQ ID NO: 1 to SEQ ID NO: 3, SEQ ID NO: 7 to SEQ ID NO: 9, or SEQ ID NO: 13 to SEQ ID NO: 15, or conservative modification forms thereof; or light chain variable region CDRs with amino acid sequences as set forth in SEQ ID NO: 4 to SEQ ID NO: 6, SEQ ID NO: 10 to SEQ ID NO: 12, or SEQ ID NO: 16 to SEQ ID NO: 18, or conservative modification forms thereof. The antibody or antigen-binding fragment according to the embodiment of the present disclosure can bind to NKp46 and promote activation of NK cells, and thus can be used to prevent or treat tumors or cancers.

In another aspect of the present disclosure, the present disclosure provides a recombinant protein. According to an embodiment of the present disclosure, the recombinant protein includes the above-described antibody or antigen-binding fragment. The recombinant protein according to the embodiments of the present disclosure can bind to NKp46 and promote activation of NK cells, and thus can be used to prevent or treat tumors or cancers.

In yet another aspect of the present disclosure, the present disclosure provides a multispecific antibody. According to an embodiment of the present disclosure, the multispecific antibody includes: a first binding region including the above-described antibody or antigen-binding fragment; and a second binding region having a binding activity to a first molecule. As can be seen from the above, the above-described antibody or antigen-binding fragment can bind to NKp46 and promote activation of NK cells. In this way, the multispecific antibody containing the above-described antibody or antigen-binding fragment can bind to NKp46 and promote activation of NK cells, and thus can be used to prevent or treat tumors or cancers.

In yet another aspect of the present disclosure, the present disclosure provides a nucleic acid molecule. According to an embodiment of the present disclosure, the nucleic acid molecule encodes the above-described antibody or antigen-binding fragment, the above-described recombinant protein, or the above-described multispecific antibody. The nucleic acid molecule according to the embodiment of the present disclosure encodes the above-described antibody or antigen-binding fragment, the above-described recombinant protein, or the above-described multispecific antibody.

In yet another aspect of the present disclosure, the present disclosure provides an expression vector. According to an embodiment of the present disclosure, the expression vector carries the above-described nucleic acid molecule. In this way, expression of the above-described antibody or antigen-binding fragment, the above-described recombinant protein, or the above-described multispecific antibody is effectively achieved, enabling large-scale *in vitro* production of the antibody or antigen-binding fragment, the recombinant protein, or the multispecific antibody.

In yet another aspect of the present disclosure, the present disclosure provides a recombinant cell. According to an embodiment of the present disclosure, the recombinant cell carries the above-described nucleic acid molecule or the above-described expression vector, or expresses the above-described antibody or antigen-binding fragment, the above-described recombinant protein, or the above-described multispecific antibody. Under suitable conditions, this recombinant cell can be used to effectively express the above-described antibody or antigen-binding fragment, recombinant protein, or multispecific antibody in the cell.

In yet another aspect of the present disclosure, the present disclosure provides a pharmaceutical composition. According to an embodiment of the present disclosure, the pharmaceutical composition includes the above-described antibody or antigen-binding fragment, the above-described recombinant protein, the above-described multispecific antibody, the above-described nucleic acid molecule, the above-described expression vector, or the above-described recombinant cell. The pharmaceutical composition of the present disclosure can bind to NKp46 and promote activation of NK cells, and thus can be used to prevent or treat tumors or cancers.

In yet another aspect of the present disclosure, the present disclosure provides a conjugate. According to an embodiment of the present disclosure, the conjugate includes: the above-described antibody or antigen-binding fragment, the above-described recombinant protein, or the above-described multispecific antibody; and a coupling moiety linked to the antibody or antigen-binding fragment, the recombinant protein, or the multispecific antibody. The conjugate of the present disclosure can bind to NKp46 and promote activation of NK cells, and thus can be used to prevent or treat tumors or cancers.

In yet another aspect of the present disclosure, the present disclosure provides a kit. According to an embodiment of the present disclosure, the kit includes: the above-described antibody or antigen-binding fragment, the above-described recombinant protein, the above-described multispecific antibody, the above-described nucleic acid molecule, the above-described expression vector, or the above-described recombinant cell. The kit of the present disclosure can effectively bind to NKp46 and can be used to effectively detect NKp46.

In yet another aspect of the present disclosure, the present disclosure provides use of the above-described antibody or antigen-binding fragment, the above-described recombinant protein, the above-described multispecific antibody, the above-described pharmaceutical composition, or the above-described conjugate in the manufacture of a medicament for preventing and/or treating an NKp46-mediated disease.

In yet another aspect of the present disclosure, the present disclosure provides use of the above-described antibody or antigen-binding fragment, the above-described recombinant protein, the above-described multispecific antibody, the above-described pharmaceutical composition, or the above-described conjugate in the prevention and/or treatment of an NKp46-mediated disease or the detection of NKp46.

In yet another aspect of the present disclosure, the present disclosure provides the above-described antibody or antigen-binding fragment, the above-described recombinant protein, the above-described multispecific antibody, the above-described pharmaceutical composition, or the above-described conjugate, for use in the prevention and/or treatment of an NKp46-mediated disease or the detection of NKp46.

In yet another aspect of the present disclosure, the present disclosure provides a method for detecting NKp46. According to an embodiment of the present disclosure, the method includes: contacting a sample to be detected with the above-described antibody or antigen-binding fragment, the above-described recombinant protein, the above-described multispecific antibody, the above-described pharmaceutical composition, the above-described conjugate, or the above-described kit to form an immune complex; and determining whether the sample to be detected contains the NKp46 or determining a content of the NKp46 based on a signal of the immune complex.

In yet another aspect of the present disclosure, the present disclosure provides a method for treating or preventing a cancer or tumor. According to an embodiment of the present disclosure, the method includes: administering to a subject a pharmaceutically acceptable amount of the above-described antibody or antigen-binding fragment, the above-described recombinant protein, the above-described multispecific antibody, the above-described pharmaceutical composition, or the above-described conjugate.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and more understandable from the following description of embodiments taken in conjunction with the accompanying drawings.
FIG. 1 shows results of a murine NKp46 antibody binding to NKp46 protein detected by ELISA in Example 2 of the present disclosure.
FIG. 2 shows results of a murine NKp46 antibody binding to PBMCs detected by flow cytometry in Example 3 of the present disclosure.
FIG. 3 shows results of a human-murine chimeric NKp46 antibody binding to NKp46 protein detected by ELISA in Example 4 of the present disclosure.

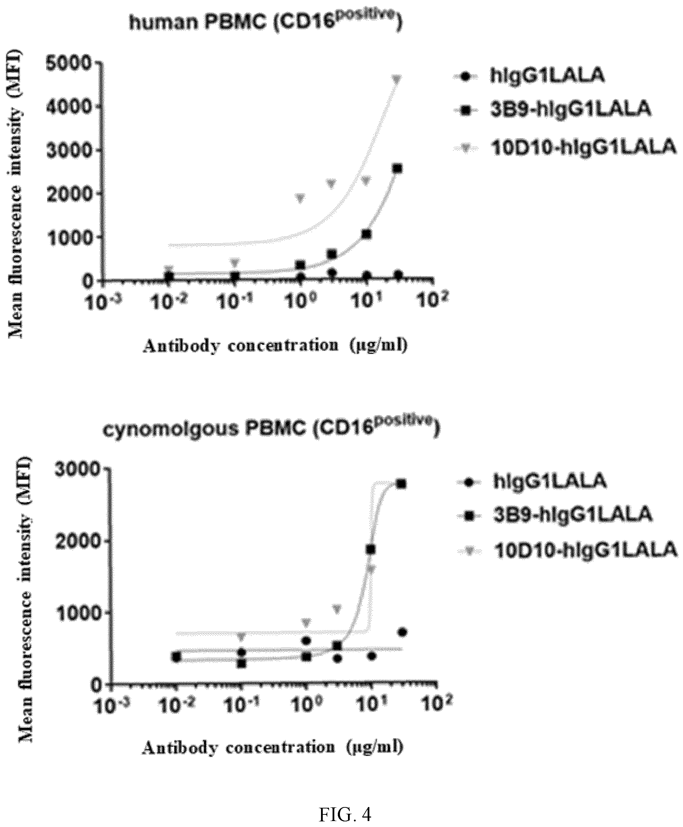

FIG. 4 shows results of a human-murine chimeric NKp46 antibody binding to PBMCs detected by flow cytometry in Example 5 of the present disclosure.
FIG. 5 shows results of a humanized NKp46 antibody binding to NKp46 protein detected by ELISA in Example 7 of the present disclosure.
FIG. 6 shows a schematic structure of a murine NKp46×MICA bispecific antibody and results of its binding to NKp46 protein detected by ELISA in Example 8 of the present disclosure.
FIG. 7 shows results of a murine NKp46×MICA bispecific antibody bridging NKp46 and MICA detected by ELISA in Example 9 of the present disclosure.
FIG. 8 shows results of a murine NKp46×MICA bispecific antibody promoting PBMCs to kill tumor cells detected by ELISA in Example 10 of the present disclosure.
FIG. 9 shows a schematic structure of a humanized NKp46×MICA bispecific antibody and results of its binding to NKp46 protein detected by ELISA in Example 11 of the present disclosure.
FIG. 10 shows results of a humanized NKp46×MICA bispecific antibody promoting PBMCs to kill tumor cells detected by ELISA in Example 12 of the present disclosure.

### DETAILED DESCRIPTION

Embodiments of the present disclosure will be described in detail below. The embodiments described below are exemplary and are only used to explain the present disclosure, and they should not be construed as limiting the present disclosure.

It should be noted that the terms "first" and "second" are only used for descriptive purposes, rather than indicating or implying relative importance or implicitly indicating the number of indicated technical features. Therefore, the features defined with "first" and "second" may include one or more of these features explicitly or implicitly. Further, in the description of the present disclosure, unless otherwise specified, "more" or "plurality" means two or more.

As used herein, the term "comprise" or "include" is an open expression, that is, including the contents indicated by the present disclosure, but not excluding other aspects.

As used herein, the terms "optionally" or "optional" generally mean that the event or situation described subsequently may but not necessarily happen, and the description includes the situation in which the event or situation happens and the situation in which the event or situation does not happen.

As used herein, the term "fragment" refers to a target protein or polypeptide, and a target protein or polypeptide with N-end (N-terminus) or C-end (C-terminus) truncation and/or internal deletion.

As used herein, a "knob-into-hole structure" refers to a knob-hole mutation formed in the CH3 region of the heavy chain constant region of the antibody to facilitate heavy chain engagement and form a heterodimer. For example, it is achieved by mutating amino acids in the CH3 domain of the human IgG1 heavy chain constant region (mutations T366S, L368A, Y407V, and Y349C in one chain as a "hole" and mutations T366W and S354C in the other chain as a "knob").

As used herein, when the term "identity", "homology", or "similarity" is a percentage of the same amino acids or nucleotides between two amino acid sequences or nucleic acid sequences determined by a conventional method, for describing an amino acid sequence or a nucleic acid sequence relative to a reference sequence.

As used herein, the term "at least 90% identity" refers to at least 90% identity to a reference sequence, which can be 90%, 91%, 92%, 93%, 94%, 95%, 96%, 97%, 98%, 99%, 99.5%, and 99.9% identity to a reference sequence.

As used herein, the amino acid numbering of the IgG1 Fc portion is numbered according to the EU numbering system. For example, position 366 refers to the 366-th position numbered according to the EU numbering system; the "T366W" means that the threonine at the 366-th position according to the EU numbering system is substituted by tryptophan; and "L368A" means that the leucine at 368-th position according to the EU numbering system is substituted by alanine.

As used herein, the term "expression vector" generally refers to a nucleic acid molecule that can be inserted into a suitable host for self-replication, to transfer the inserted nucleic acid molecule into and/or between host cells. The expression vector may include a vector mainly used to insert DNA or RNA into cells, a vector mainly used to replicate DNA or RNA, and a vector mainly used to express, i.e., transcript and/or translate, DNA or RNA. The expression vector also includes vectors with multiple functions as described above. The expression vector may be a polynucleotide that can be transcribed and translated into a polypeptide when introduced into a suitable host cell. Generally, the expression vector can produce a desired expression product by culturing the appropriate host cell containing the expression vector.

As used herein, the term "recombinant cell" usually refers to a cell with stably inherited unique traits that are obtained by modifying or recombining the genetic material of a host cell using genetic engineering technology or cell fusion technology. The term "host cell" refers to a prokaryotic cell or a eukaryotic cell, into which a recombinant expression vector can be introduced. As used herein, the term "transformed" or "transfected" refers to the introduction of a nucleic acid (such as a vector) into a cell by various techniques known in the art. Suitable host cells can be transformed or transfected with the DNA sequence of the present disclosure, and can be used for the expression and/or secretion of target proteins. Examples of suitable host cells that can be used in the present disclosure include immortalized hybridoma cells, NS/0 myeloma cells, 293 cells, Chinese hamster ovary (CHO) cells, HeLa cells, Cap cells (cells derived from human amniotic fluid), and CoS cells.

As used herein, the term "pharmaceutical composition" generally refers to a unit dosage form and can be prepared by any method well known in the pharmaceutical field. All the methods include the step of combining the active ingredient with a carrier, which constitutes one or more accessory ingredients. Generally, a composition is prepared by uniformly and sufficiently combining the active antibody or antigen-binding fragment with a liquid carrier, a finely divided solid carrier or both.

As used herein, the term "pharmaceutically acceptable excipient" may include any solvent, solid excipient, diluent or other liquid excipient, etc., which is suitable for the specific target dosage form. Except to the extent that any conventional excipients are incompatible with the antibody or antigen-binding fragment of the present disclosure, for example, producing any adverse biological effects or harmful interactions with any other components of a pharmaceutically acceptable composition, their uses are also contemplated by the present disclosure.

As used herein, the term "administrating" refers to introducing a predetermined amount of substance into a patient by a certain suitable route. The antibody or antigen-binding fragment, the recombinant protein, the multispecific antibody, or the pharmaceutical composition of the present disclosure can be administered by any common route as long as it can reach the intended tissue. Various administration routes can be contemplated, including peritoneal injection, intravenous injection, intramuscular injection, subcutaneous injection, etc. However, the present disclosure is not limited to these exemplified administration routes. Preferably, the composition of the present disclosure is administered by intravenous injection or subcutaneous injection.

As used herein, the term "treatment" or "treat" refers to obtaining a desired pharmacological and/or physiological effect. The effect may be preventive in terms of completely or partially preventing the disease or its symptoms, and/or therapeutic in terms of partially or completely curing the disease and/or adverse effects caused by the disease. As used herein, the term "treatment" or "treat" is intend to aim at diseases in mammals, especially humans, including: (a) preventing the occurrence of diseases or disorders in individuals who are susceptible to diseases but have not been diagnosed with the diseases; (b) inhibiting diseases, for example, retarding the progression of the diseases; or (c) alleviating diseases, such as alleviating the symptoms associated with the diseases. As used herein, the term "treatment" or "treat" includes treating, curing, alleviating, ameliorating, mitigating or inhibiting disease in an individual by administrating any of a medicament or an antibody or antigen-binding fragment to the individual, including but not limited to, administering a medicament containing the antibody or antigen-binding fragment described herein to an individual in need thereof.

The present disclosure provides an antibody or antigen-binding fragment, a recombinant protein, a multispecific antibody, a nucleic acid molecule, an expression vector, a recombinant cell, a pharmaceutical composition, a conjugate, a kit, and use thereof, which will be described in detail below.

### Antibody or Antigen-Binding Fragment

In a first aspect of the present disclosure, the present disclosure provides an antibody or antigen-binding fragment. According to an embodiment of the present disclosure, the antibody or antigen-binding fragment includes a CDR selected from at least one of: heavy chain variable region CDRs with amino acid sequences as set forth in SEQ ID NO: 1 to SEQ ID NO: 3, SEQ ID NO: 7 to SEQ ID NO: 9, or SEQ ID NO: 13 to SEQ ID NO: 15, or conservative modification forms thereof; or light chain variable region CDRs with amino acid sequences as set forth in SEQ ID NO: 4 to SEQ ID NO: 6, SEQ ID NO: 10 to SEQ ID NO: 12, or SEQ ID NO: 16 to SEQ ID NO: 18, or conservative modification forms thereof. The antibody or antigen-binding fragment according to the embodiment of the present disclosure can bind to NKp46 and promote activation of NK cells, and thus can be used to prevent or treat tumors or cancers.

As used herein, the term "antibody" is used in the broadest sense, and it may include full-length monoclonal antibodies, multi-specific antibodies, and chimeric antibodies. The specific structure is not limited as long as they exhibit the desired biological activity. The antibody usually includes a light chain with a lighter molecular weight and a heavy chain with a heavier molecular weight. The heavy chain (H chain) and the light chain (L chain) are connected by disulfide bonds to form an antibody molecule. The amino acid sequence of the amino terminal (N terminal) of the peptide chain varies significantly, which is referred to as a variable region (V region), while the carboxyl terminal (C terminal) is relatively stable and varies insignificantly, which is referred to as a constant region (C region). The V region of the L chain and the V region of the H chain are referred to as VL and VH, respectively. As used herein, the terms "complementarity determining region", "CDR", or "CDRs" refer to highly variable regions of the heavy chain and the light chain of immunoglobulins, and refer to the regions containing one or more or even all of the main amino acid residues of the antibody or the functional fragment thereof that contribute to the binding affinity for recognized antigen or epitope. In a specific embodiment of the present disclosure, CDRs refer to highly variable regions of the heavy chain and the light chain of the antibody.

As used herein, the term "antigen-binding fragment" refers to a fragment including part or all of an antibody, while lacking at least some of the amino acids present in the full-length chain but is still capable of specifically binding to an antigen. For example, the fragment may include part or all of the CDRs of the antibody. Such fragments are biologically active because they bind to an antigen and can compete with other antigen-binding molecules (including intact antibodies) for binding to a given epitope. Such fragments are selected from Fab, Fv, scFv, or single-domain antibodies. Such fragments can be produced by recombinant nucleic acid technology, or can be produced by enzymatic cleavage or chemical cleavage of antigen-binding molecules (including intact antibodies).

As used herein, a "conservative modification form of an amino acid sequence" refers to an amino acid modification that does not significantly affect or alter the binding characteristics of an antibody comprising the amino acid sequence, including amino acid substitutions, additions, and deletions. Modifications can be introduced into the antibody of the present disclosure by standard techniques such as site-directed mutagenesis and PCR-mediated mutagenesis. Conservative amino acid substitution refers to the substitution of amino acid residues by amino acid residues with similar side chains. Families of amino acid residues having similar side chains have been identified in the art. These families include amino acids with basic side chains, e.g. lysine, arginine, histidine, amino acids with acidic side chains, e.g. aspartic acid, glutamic acid, amino acids with uncharged polar side chains, e.g. glycine, asparagine, glutamine, serine, threonine, tyrosine, cysteine, tryptophan, amino acids with nonpolar side chains, e.g. alanine, valine, leucine, isoleucine, proline, phenylalanine, methionine, amino acids with β-branched side chains, e.g. threonine, valine, isoleucine, and amino acids with aromatic side chains, e.g. tyrosine, phenylalanine, tryptophan, histidine. Thus, one or more of the amino acid residues in the CDRs of an antibody of the present disclosure can be substituted with other amino acid residues from the same side chain family, and the altered antibody can be tested for retained function using the functional assays described herein. Preferably, the number of conservative modifications does not exceed one or two.

According to an embodiment of the present disclosure, the above-described antibody or antigen-binding fragment may further include at least one of the following additional technical features.

According to an embodiment of the present disclosure, the antibody or antigen-binding fragment includes: heavy chain variable region CDR1, heavy chain variable region CDR2, and heavy chain variable region CDR3 with an amino acid sequence each set forth in SEQ ID NO: 1, SEQ ID NO: 2, and SEQ ID NO: 3; or heavy chain variable region CDR1, heavy chain variable region CDR2, and heavy chain variable region CDR3 with an amino acid sequence each set forth in SEQ ID NO: 7, SEQ ID NO: 8, and SEQ ID NO: 9; or heavy chain variable region CDR1, heavy chain variable region CDR2, and heavy chain variable region CDR3 with an amino acid sequence each set forth in SEQ ID NO: 13, SEQ ID NO: 14, and SEQ ID NO: 15.

According to an embodiment of the present disclosure, the antibody or antigen-binding fragment includes: light chain variable region CDR1, light chain variable region CDR2, and light chain variable region CDR3 with an amino acid sequence each set forth in SEQ ID NO: 4, SEQ ID NO: 5, and SEQ ID NO: 6; or light chain variable region CDR1, light chain variable region CDR2, and light chain variable region CDR3 with an amino acid sequence each set forth in set forth in SEQ ID NO: 10, SEQ ID NO: 11, and SEQ ID NO: 12; or light chain variable region CDR1, light chain variable region CDR2, and light chain variable region CDR3 with an amino acid sequence each set forth in SEQ ID NO: 16, SEQ ID NO: 17, and SEQ ID NO: 18.

According to an embodiment of the present disclosure, the antibody or antigen-binding fragment includes: heavy chain variable region CDR1, heavy chain variable region CDR2, and heavy chain variable region CDR3 with an amino acid sequence each set forth in SEQ ID NO: 1, SEQ ID NO: 2, and SEQ ID NO: 3, and light chain variable region CDR1, light chain variable region CDR2, and light chain variable region CDR3 with an amino acid sequence each set forth in SEQ ID NO: 4, SEQ ID NO: 5, and SEQ ID NO: 6; or heavy chain variable region CDR1, heavy chain variable region CDR2, and heavy chain variable region CDR3 with an amino acid sequence each set forth in SEQ ID NO: 7, SEQ ID NO: 8, and SEQ ID NO: 9, and light chain variable region CDR1, light chain variable region CDR2, and light chain variable region CDR3 with an amino acid sequence each set forth in SEQ ID NO: 10, SEQ ID NO: 11, and SEQ ID NO: 12; or heavy chain variable region CDR1, heavy chain variable region CDR2, and heavy chain variable region CDR3 with an amino acid sequence each set forth in SEQ ID NO: 13, SEQ ID NO: 14, and SEQ ID NO: 15, and light chain variable region CDR1, light chain variable region CDR2, and light chain variable region CDR3 with an amino acid sequence each set forth in SEQ ID NO: 16, SEQ ID NO: 17, and SEQ ID NO: 18.

According to an embodiment of the present disclosure, the antibody or antigen-binding fragment includes a heavy chain framework region and/or a light chain framework region.

According to an embodiment of the present disclosure, at least a portion of the heavy chain framework region and/or the light chain framework region is derived from at least one of a murine antibody, a human antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof, preferably at least one of a murine antibody, a human antibody, or a primate antibody.

According to an embodiment of the present disclosure, the antibody or antigen-binding fragment includes: a heavy chain variable region with an amino acid sequence set forth in any one of SEQ ID NO: 19, SEQ ID NO: 21, SEQ ID NO: 23, and SEQ ID NO: 25, or an amino acid sequence having at least 90% homology thereto; and/or a light chain variable region with an amino acid sequence set forth in any one of SEQ ID NO: 20, SEQ ID NO: 22, SEQ ID NO: 24, and SEQ ID NO: 26, or an amino acid sequence having at least 90% homology thereto.

According to an embodiment of the present disclosure, the antibody or antigen-binding fragment includes: a heavy chain variable region with an amino acid sequence set forth in SEQ ID NO: 19 or an amino acid sequence having at least 90% homology thereto, and a light chain variable region with an amino acid sequence set forth in any one of SEQ ID NO: 20 or an amino acid sequence having at least 90% homology thereto; or a heavy chain variable region with an amino acid sequence set forth in SEQ ID NO: 21 or an amino acid sequence having at least 90% homology thereto, and a light chain variable region with an amino acid sequence set forth in any one of SEQ ID NO: 22 or an amino acid sequence having at least 90% homology thereto; or a heavy chain variable region with an amino acid sequence set forth in SEQ ID NO: 23 or an amino acid sequence having at least 90% homology thereto, and a light chain variable region with an amino acid sequence set forth in any one of SEQ ID NO: 24 or an amino acid sequence having at least 90% homology thereto; or a heavy chain variable region with an amino acid sequence set forth in SEQ ID NO: 25 or an amino acid sequence having at least 90% homology thereto, and a light chain variable region with an amino acid sequence set forth in any one of SEQ ID NO: 26 or an amino acid sequence having at least 90% homology thereto.

According to an embodiment of the present disclosure, the antibody or antigen-binding fragment further includes a constant region. The constant region includes at least one of a heavy chain constant region and a light chain constant region.

According to an embodiment of the present disclosure, at least a portion of at least one of the heavy chain constant region and the light chain constant region is derived from at least one of a murine antibody, a human antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof.

According to an embodiment of the present disclosure, the heavy chain constant region includes a heavy chain constant region selected from IgG1, IgG2, IgG3, IgG4, IgA, IgM, IgE, or IgD; or the light chain constant region includes a light chain constant region selected from a x-type light chain constant region or a λ-type light chain constant region.

According to an embodiment of the present disclosure, the light chain constant region and the heavy chain constant region are both derived from a murine antibody or a mutant thereof, or a human antibody or a mutant thereof.

According to an embodiment of the present disclosure, the N-terminus of the heavy chain constant region is linked to the C-terminus of the heavy chain variable region; and/or the N-terminus of the light chain constant region is linked to the N-terminus of the light chain variable region.

According to an embodiment of the present disclosure, the heavy chain constant region includes a heavy chain constant region with an amino acid sequence set forth in SEQ ID NO: 27 or an amino acid sequence having at least 80% identity thereto, or a heavy chain constant region with an amino acid sequence set forth in SEQ ID NO: 28 or an amino acid sequence having at least 80% identity thereto; and/or the light chain constant region includes or is a light chain constant region with an amino acid sequence set forth in SEQ ID NO: 55 or an amino acid sequence having at least 80% identity thereto, or the light chain constant region includes or is a light chain constant region with an amino acid sequence set forth in SEQ ID NO: 56 or an amino acid sequence having at least 80% identity thereto.

According to an embodiment of the present disclosure, the antibody or antigen-binding fragment includes: a heavy chain with an amino acid sequence set forth in any one of SEQ ID NO: 29, SEQ ID NO: 31, SEQ ID NO: 33, SEQ ID NO: 35, SEQ ID NO: 37, SEQ ID NO: 39, and SEQ ID NO: 41, or an amino acid sequence having at least 90% homology thereto; and/or a light chain with an amino acid sequence set forth in any one of SEQ ID NO: 30, SEQ ID NO: 32, SEQ ID NO: 34, SEQ ID NO: 36, SEQ ID NO: 38, SEQ ID NO: 40, SEQ ID NO: 42, or an amino acid sequence having at least 90% homology thereto.

According to an embodiment of the present disclosure, the antibody or antigen-binding fragment includes: a heavy chain with an amino acid sequence set forth in SEQ ID NO: 29 or an amino acid sequence having at least 80% homology thereto, and a light chain with an amino acid sequence set forth in SEQ ID NO: 30 or an amino acid sequence having at least 80% homology thereto; or a heavy chain with an amino acid sequence set forth in SEQ ID NO: 31 or an amino acid sequence having at least 80% homology thereto, and a light chain with an amino acid sequence set forth in SEQ ID NO: 32 or an amino acid sequence having at least 80% homology thereto; or a heavy chain with an amino acid sequence set forth in SEQ ID NO: 33 or an amino acid sequence having at least 80% homology thereto, and a light chain with an amino acid sequence set forth in SEQ ID NO: 34 or an amino acid sequence having at least 80% homology thereto; or a heavy chain with an amino acid sequence set forth in SEQ ID NO: 35 or an amino acid sequence having at least 80% homology thereto, and a light chain with an amino acid sequence set forth in SEQ ID NO: 36 or an amino acid sequence having at least 80% homology thereto; or a heavy chain with an amino acid sequence set forth in SEQ ID NO: 37 or an amino acid sequence having at least 80% homology thereto, and a light chain with an amino acid sequence set forth in SEQ ID NO: 38 or an amino acid sequence having at least 80% homology thereto; or a heavy chain with an amino acid sequence set forth in SEQ ID NO: 39 or an amino acid sequence having at least 80% homology thereto, and a light chain with an amino acid sequence set forth in SEQ ID NO: 40 or an amino acid sequence having at least 80% homology thereto; or a heavy chain with an amino acid sequence set forth in SEQ ID NO: 41 or an amino acid sequence having at least 80% homology thereto, and a light chain with an amino acid sequence set forth in SEQ ID NO: 42 or an amino acid sequence having at least 80% homology thereto.

According to an embodiment of the present disclosure, the antibody includes at least one selected from a polyclonal antibody, a full-length monoclonal antibody, a Fab antibody, a Fab' antibody, a F(ab')₂ antibody, a Fv antibody, a single-chain antibody, a single-domain antibody, and a minimum recognition unit; or the antigen-binding fragment includes at least one selected from a F(ab')₂ fragment, a Fab' fragment, a Fab fragment, a F(ab)₂ fragment, a Fv fragment, a scFv fragment, a scFv-Fc fusion protein, a scFv-Fv fusion protein, and a minimum recognition unit.

As used herein, the terms "full-length antibody", "full-length mAb", or "full-length monoclonal antibody" are all composed of at least two identical light chains and at least two identical heavy chains connected by interchain disulfide bonds, such as immunoglobulin G (IgG), immunoglobulin A (IgA), immunoglobulin M (IgM), immunoglobulin D (IgD), or immunoglobulin E (IgE).

As used herein, the term "multispecific antibody" refers to an antibody capable of recognizing multiple antigenic epitopes, such as an antibody capable of recognizing two antigenic epitopes (bispecific antibody, abbreviated as BsAb), an antibody capable of recognizing three antigenic epitopes, or an antibody capable of recognizing four antigenic epitopes. The terms are understood in a broad sense and specific structures thereof are not limited, as long as multiple antigenic epitopes can be recognized. In the present disclosure, at least one of the multiple antigenic epitopes is derived from NKp46.

As used herein, the terms "single domain antibody", "nanobody", and "VHH antibody" are used interchangeably. The terms were originally described as the antigen-binding immunoglobulin (variable) domain of a "heavy chain antibody" (i.e. "an antibody devoid of a light chain") (Hamers-Casterman C, AtarhouchT, Muyldermans S, Robinson G, Hamers C, Songa EB, Bendahman N, Hamers R .:"Naturallyoccurring antibodies devoid of light chains"; Nature 363 ,446-448(1993)), which includes a heavy chain variable region (VH) and conventional CH2 and CH3 regions, and specifically binds to an antigenic protein (such as NKp46) through the variable region of the heavy chain.

As used herein, the term "Fab antibody" or "Fab fragment" usually refers to an antibody or fragment containing only Fab molecule, which is composed of VH and CH1 of the heavy chain and the intact light chain. The light chain and the heavy chain are connected by one disulfide bond.

As used herein, the term "F(ab')₂ antibody" or "F(ab')₂ fragment" has two antigen-binding F(ab') portions linked together by disulfide bonds.

As used herein, the term "Fv antibody" or "Fv fragment" usually refers to an antibody or a fragment only composed of the light chain variable region (VL) and the heavy chain variable region (VH) connected by non-covalent bonds, and it is the smallest functional fragment of the antibody that retains the complete antigen-binding site.

As used herein, the terms "single-chain antibody" and "scFv fragment" refer to antibodies or fragments composed of the heavy chain variable region and the light chain variable region of the antibody that are connected via a short peptide.

As used herein, the terms "minimum recognition unit" and "MRU" both refer to antibodies or fragments composed of only one CDR, with an extremely small molecular weight that accounts for only about 1% of the complete antibody.

### Recombinant Protein

In another aspect of the present disclosure, the present disclosure provides a recombinant protein. According to an embodiment of the present disclosure, the recombinant protein includes the above-described antibody or antigen-binding fragment. The recombinant protein according to the embodiment of the present disclosure can bind to NKp46 and promote activation of NK cells, and thus can be used to prevent or treat tumors or cancers.

According to an embodiment of the present disclosure, the recombinant protein further includes at least one of a biologically active protein or a fragment thereof, or a biologically active polypeptide or a fragment thereof.

According to an embodiment of the present disclosure, the biologically active protein or the fragment thereof includes at least one selected from a protein tag, a protein toxin or a fragment thereof, a tumor necrosis factor or a fragment thereof, an interferon or a fragment thereof, a biological response modifier or a fragment thereof, or an Fc fragment.

As used herein, "protein tag" generally refers to a polypeptide or protein that is fused and expressed with a target protein (the antibody or antigen-binding fragment), and can be used for expression, detection, tracing, or purification of the target protein, etc. The protein tag includes, but is not limited to, His tag (with a sequence of HHHHHH), Flag tag (with a sequence of DYKDDDDK), GST tag (glutathione sulfhydryl transferase tag), MBP tag (maltose binding protein tag), SUMO tag, and C-Myc tag.

As used herein, "toxin" generally refers to a substance toxic to the host, including protein toxins and non-protein toxins. Protein toxins include, but are not limited to, abrin, ricin A, Pseudomonas exotoxin, and diphtheria toxin. In the present disclosure, the protein toxin is preferably a protein toxin with enzymatic activity.

As used herein, "tumor necrosis factor" generally refers to substances that can cause hemorrhagic necrosis in various tumors, including but not limited to TNF-α and TNF-β.

As used herein, "interferon" generally refers to a glycoprotein with direct killing or inhibitory effects on viruses, including but not limited to IFN-α, INF-β, and IFN-γ.

As used herein, "biological response regulator" generally refers to a class of protein substances that directly or indirectly enhance the body's anti-tumor effects through the immune system, including but not limited to lymphokines, IL-2, IL-6, IL-10, and GM-CSF.

As used herein, "Fc fragment" generally refers to an Fc region from IgG (such as IgG1, IgG2, IgG3, or IgG4 subtypes), IgA1, IgA2, IgD, IgE, or IgM, including the CH2, CH3 regions and optionally the hinge region. Preferably, IgG, IgA1, IgA2, IgD, IgE, or IgM is derived from mouse/rat, human, primate, or alpaca.

### Multispecific Antibody

In yet another aspect of the present disclosure, the present disclosure provides a multispecific antibody. According to an embodiment of the present disclosure, the multispecific antibody includes: a first binding region, including the above-described antibody or antigen-binding fragment; and a second binding region, having a binding activity to a first molecule. As can be seen from the above, the above-described antibody or antigen-binding fragment can bind to NKp46 and promote activation of NK cells, and thus can be used to prevent or treat tumors or cancers. In this way, the multispecific antibody containing the above-described antibody or antigen-binding fragment can bind to NKp46 and promote the activation of NK cells, and thus can be used to prevent or treat tumors or cancers.

According to an embodiment of the present disclosure, the multispecific antibody further includes at least one of the following additional technical features.

According to an embodiment of the present disclosure, the multispecific antibody includes at least one selected from a bispecific antibody, a trispecific antibody, or a tetraspecific antibody, preferably the bispecific antibody.

It should be noted that, the second binding region may be one or more. In the case of bispecific antibody, one second binding region is provided. In the case of trispecific antibody, two second binding regions are provided, and the two second binding regions may bind to two different first molecules, respectively. In the case of tetraspecific antibody, three second binding region are provided, and the three second binding regions may bind to three different first molecules, respectively.

According to an embodiment of the present disclosure, the first molecule includes at least one selected from a tumor-associated antigen, a virus, a bacterium, an endotoxin, a cytokine, or a cytokine receptor, preferably a tumor antigen.

As used herein, "tumor-associated antigen" should be understood in a broad sense, and may refer to a protein that is differentially expressed in tumor cells compared to normal tissue cells. Examples include, but are not limited to, Her2, EpCAM, PD-L1, CEA, GD2, and GD3.

As used herein, "cytokine" should be understood in a broad sense, and may refer to a class of proteins or small molecular polypeptides that can transmit information between cells and have immunomodulatory and effector functions. Examples include, but are not limited to, IL-10, VEGF, EpCAM, GM2, and RANKL.

As used herein, "cytokine receptor" should be understood in a broad sense, and may refer to a receptor on the cell surface that can bind to cytokines. Examples include, but are not limited to, Her2, EGFR and IL-10R.

According to an embodiment of the present disclosure, the first molecule includes, but is not limited to, B7H6, MICA, MICB, and PDL1, preferably MICA.

According to an embodiment of the present disclosure, the second binding region is a binding protein or a fragment thereof to the first molecule.

It should be noted that, "binding protein or a fragment thereof to the first molecule" generally refers to a protein or a fragment thereof that can bind to the first molecule. In the case that the first molecule is a protein receptor, the binding protein or the fragment thereof to the first molecule may be a cytokine or a fragment thereof, or an antibody or a fragment thereof that binds to the protein receptor. In the case that the first molecule is a protein other than a protein receptor, the binding protein or the fragment thereof to the first molecule may be a protein receptor or a fragment thereof, or an antibody or a fragment thereof that binds to the protein.

According to an embodiment of the present disclosure, the first binding region includes a first heavy chain variable region and a first light chain variable region. The C-terminus of the first heavy chain variable region is linked to the N-terminus of the first light chain variable region; or the N-terminus of the first heavy chain variable region is linked to the C-terminus of the first light chain variable region.

According to an embodiment of the present disclosure, the first binding region further includes a first linking peptide. The C-terminus of the first heavy chain variable region is linked to an N-terminus of the first linking peptide, and a C-terminus of the first linking peptide is linked to the N-terminus of the first light chain variable region; or the C-terminus of the first light chain variable region is linked to the N-terminus of the first linking peptide, and the C-terminus of the first linking peptide is linked to the N-terminus of the first heavy chain variable region.

According to an embodiment of the present disclosure, the first heavy chain variable region is a heavy chain variable region characterized by the above-described antibody or antigen-binding fragment; and/or the first light chain variable region is a light chain variable region characterized by the above-described antibody or antigen-binding fragment.

According to an embodiment of the present disclosure, the first binding region further includes a first Fc fragment. The C-terminus of the first heavy chain variable region is linked to the N-terminus of the first linking peptide, the C-terminus of the first linking peptide is linked to the N-terminus of the first light chain variable region, and the C-terminus of the first light chain variable region is linked to an N-terminus of the first Fc fragment; or the C-terminus of the first light chain variable region is linked to the N-terminus of the first linking peptide, the C-terminus of the first linking peptide is linked to the N-terminus of the first heavy chain variable region, and the C-terminus of the first heavy chain variable region is linked to the N-terminus of the first Fc fragment.

According to an embodiment of the present disclosure, relative to an amino acid sequence of an Fc fragment from a wild-type human IgG1, the first Fc fragment has at least one mutation site selected from: C220S, L234A, L235A, T366W, and S354C.

According to an embodiment of the present disclosure, the first Fc fragment has an amino acid sequence set forth in SEQ ID NO: 43.

According to an embodiment of the present disclosure, the first binding region further includes a second linking peptide.

According to an embodiment of the present disclosure, the N-terminus of the second linking peptide is linked to the C-terminus of the first light chain variable region, and the C-terminus of the second linking peptide is linked to the N-terminus of the first Fc fragment; or the N-terminus of the second linking peptide is linked to the C-terminus of the first heavy chain variable region, and the C-terminus of the second linking peptide is linked to the N-terminus of the first Fc fragment.

According to an embodiment of the present disclosure, an amino acid sequence of the first linking peptide and/or the second linking peptide is (GGGGS)ₙ, where n is an integer greater than or equal to 1, preferably 1, 2, 3, 4, 5, 6, 7, 8, 9, or 10.

According to an embodiment of the present disclosure, the first linking peptide has an amino acid sequence set forth in SEQ ID NO: 44.

According to an embodiment of the present disclosure, the second linking peptide is GGGGS (SEQ ID NO: 68).

According to an embodiment of the present disclosure, the first binding region includes an amino acid sequence set forth in any one of SEQ ID NO: 45, SEQ ID NO: 47, SEQ ID NO: 57, and SEQ ID NO: 59.

According to an embodiment of the present disclosure, the first binding region has an amino acid sequence set forth in any one of SEQ ID NO: 46, SEQ ID NO: 48, SEQ ID NO: 58, and SEQ ID NO: 60.

According to an embodiment of the present disclosure, the second binding region includes a first polypeptide and a second polypeptide. The first polypeptide and the second polypeptide are linked via an interchain disulfide bond. The first polypeptide includes a second heavy chain variable region, a CH1 region, and a second Fc fragment. The C-terminus of the second heavy chain variable region is linked to the N-terminus of the CH1 region, and the C-terminus of the CH1 region is linked to the N-terminus of the second Fc fragment. The second polypeptide includes a second light chain variable region and a CL region. The C-terminus of the second light chain variable region is linked to the N-terminus of the CL region.

According to an embodiment of the present disclosure, the CH1 region includes a CH1 region selected from a wild-type human IgG1, a wild-type primate IgG1, or a wild-type murine IgG1, preferably the CH1 region of the wild-type human IgG1.

Exemplarily, the CH1 region of the wild-type human IgG1 has an amino acid sequence set forth in SEQ ID NO: 66.

According to an embodiment of the present disclosure, the second Fc fragment, relative to an amino acid sequence of an Fc fragment from a wild-type human IgG1, has at least one mutation site selected from: L234A, L235A, Y349C, T366S, L368A, and Y407V.

According to an embodiment of the present disclosure, the second Fc fragment has an amino acid sequence set forth in SEQ ID NO: 49.

According to an embodiment of the present disclosure, the CL region is a wild-type human CL region, a wild-type primate CL region, or a wild-type murine CL region, preferably the wild-type human CL region.

According to an embodiment of the present disclosure, the wild-type human CL region has an amino acid sequence set forth in SEQ ID NO: 67.

According to an embodiment of the present disclosure, the second heavy chain variable region has an amino acid sequence set forth in SEQ ID NO: 51.

According to an embodiment of the present disclosure, the second light chain variable region has an amino acid sequence set forth in SEQ ID NO: 52.

According to an embodiment of the present disclosure, the first polypeptide has an amino acid sequence set forth in SEQ ID NO: 53.

According to an embodiment of the present disclosure, wherein the second polypeptide has an amino acid sequence set forth in SEQ ID NO: 54.

According to an embodiment of the present disclosure, the first binding region and the second binding region are linked via a knob-into-hole structure.

### Nucleic Acid Molecule, Expression Vector, And Recombinant Cell

In yet another aspect of the present disclosure, the present disclosure provides a nucleic acid molecule. According to an embodiment of the present disclosure, the nucleic acid molecule, encodes the above-described antibody or antigen-binding fragment, the above-described recombinant protein, or the above-described multispecific antibody. The nucleic acid molecule according to the embodiment of the present disclosure, encodes the above-described antibody or antigen-binding fragment, the above-described recombinant protein, or the above-described multispecific antibody.

According to an embodiment of the present disclosure, the nucleic acid molecule is DNA.

It should be noted that, for the nucleic acid molecules mentioned herein, those skilled in the art should understand that they actually include any one or two of the complementary double strands. In this specification and claims, for convenience, only one strand is given in most cases, but the other strand complementary to the one strand is actually also disclosed. In addition, the nucleic acid sequences in the present disclosure include a DNA form or an RNA form, the disclosure of one of which implies the disclosure of the other.

In yet another aspect of the present disclosure, the present disclosure provides an expression vector. According to an embodiment of the present disclosure, the expression vector carries the above-described nucleic acid molecule. When the above-described nucleic acid molecule is connected to the vector, the nucleic acid molecule can be directly or indirectly connected to control elements on the vector, as long as these control elements can control the translation, expression and the like of the nucleic acid molecule. These control elements may be directly derived from the vector itself, or they may be exogenous, i.e., not derived from the vector itself. The nucleic acid molecule is operably connected to the control elements. As used herein, "operably connected" means that an exogenous gene is connected to the vector, in such a manner that the control elements in the vector, such as transcription control sequences and translation control sequences, can play their expected functions of regulating the transcription and translation of the exogenous gene. Common vectors may be, for example, plasmids, bacteriophages, etc. After the expression vector according to some specific embodiments of the present disclosure is introduced into suitable recipient cells, the expression of the above-described antibody or the antigen-binding fragment thereof, the above-described recombinant protein, or the above-described multispecific antibody can be effectively achieved under the mediation of the regulatory system, enabling large-scale *in vitro* production of the antibodies or the antigen-binding fragments thereof, the recombinant proteins, or the multispecific antibodies.

According to an embodiment of the present disclosure, the expression vector is a eukaryotic expression vector or a prokaryotic expression vector.

According to an embodiment of the present disclosure, the expression vector is a plasmid expression vector.

In yet another aspect of the present disclosure, the present disclosure provides a recombinant cell. According to an embodiment of the present disclosure, the recombinant cell carries the above-described nucleic acid molecule or the above-described expression vector, or expresses the above-described antibody or antigen-binding fragment, the above-described recombinant protein, or the above-described multispecific antibody. Under suitable conditions, this recombinant cell can be used to effectively express the above-described antibody or antigen-binding fragment thereof, the above-described recombinant protein, or the above-described multispecific antibody in the cell.

It should be noted that, "suitable conditions" described in the specification of the present disclosure refer to conditions suitable for the expression of the antibody or the antigen-binding fragment thereof, the recombinant protein, or the multispecific antibody described in the present disclosure. It is conceivable for those skilled in the art that the conditions suitable for the expression of the antibody or the antigen-binding fragment thereof, the recombinant protein, or the multispecific antibody include, but are not limited to, appropriate transformation or transfection methods, appropriate transformation or transfection conditions, healthy host cell states, appropriate host cell density, suitable cell culture environment, and suitable cell culture time. The "suitable conditions" are not particularly limited, and those skilled in the art can optimize the most suitable conditions for the expression of the antibody or the antigen-binding fragment thereof, the recombinant protein, or the multispecific antibody according to the specific environment of the laboratory.

According to an embodiment of the present disclosure, the recombinant cell is obtained by introducing the above-described expression vector into a host cell.

According to an embodiment of the present disclosure, the recombinant cell is a eukaryotic cell, preferably a mammalian cell.

### Pharmaceutical Composition, Conjugate, And Kit

In yet another aspect of the present disclosure, the present disclosure provides a pharmaceutical composition. According to an embodiment of the present disclosure, the pharmaceutical composition includes the above-described antibody or antigen-binding fragment, the above-described recombinant protein, the above-described multispecific antibody, the above-described nucleic acid molecule, the above-described expression vector, or the above-described recombinant cell. As can be seen from the above, the above-described antibody or antigen-binding fragment can bind to NKp46, and the multispecific antibody containing the above-described antibody or antigen-binding fragment can bind to NKp46. In this way, the pharmaceutical composition containing the above-described antibody or antigen-binding fragment and multispecific antibody can also bind to NKp46 and promote activation of NK cells, and thus can be used to prevent or treat tumors or cancers.

According to an embodiment of the present disclosure, the pharmaceutical composition further includes a pharmaceutically acceptable excipient.

The administration of the pharmaceutical composition of the present disclosure can be carried out by any acceptable administration mode. The pharmaceutical composition of the present disclosure can be formulated into formulations in solid, semi-solid, liquid, or gaseous forms, such as injections and freeze-dried powders. Current methods for preparing these dosage forms are known or obvious to those skilled in the art. Typical routes for administering such pharmaceutical compositions include, but are not limited to, oral, topical, transdermal, inhalation, parenteral, sublingual, buccal, rectal, vaginal, and intranasal routes. The term "parenteral" as used herein includes subcutaneous injection, intravenous, intramuscular, intradermal, intrasternal injection or infusion techniques. The pharmaceutical composition of the present disclosure is formulated to allow the biologically active ingredients contained therein to be bioavailable after the composition is administered to a patient.

In yet another aspect of the present disclosure, the present disclosure provides a conjugate. According to an embodiment of the present disclosure, the conjugate includes: the above-described antibody or antigen-binding fragment, the above-described recombinant protein, or the above-described multispecific antibody; and a coupling moiety, linked to the antibody or antigen-binding fragment, the recombinant protein, or the multispecific antibody. As can be seen from the above, the above-described antibody or antigen-binding fragment can bind to NKp46, and the multispecific antibody containing the above-described antibody or antigen-binding fragment can bind to NKp46. In this way, the conjugate containing the above-described antibody or antigen-binding fragment and multispecific antibody can also bind to NKp46 and promote activation of NK cells, and thus can be used to prevent or treat tumors or cancers.

According to an embodiment of the present disclosure, the coupling moiety includes at least one selected from a carrier, a drug, a toxin, a cytokine, a protein tag, a modifier, and a chemotherapeutic agent.

As used herein, the carrier may be a substance that can be suspended or dispersed in a liquid phase (e.g., a solid-phase carrier such as a particle and a magnetic bead), or a solid phase that can contain or carry a liquid phase (e.g., a support such as a plate, a membrane, and a test tube, as well as a container such as a well plate, a microfluidic channel, a glass capillary, a nanocolumn, and a monolithic column). The carrier may also be a labeling carrier used to label an antibody or antigen-binding fragment, a recombinant protein, or a multispecific antibody, such as an enzyme (e.g., peroxidase, alkaline phosphatase, luciferin, and β-galactosidase), an affinity substance (e.g., one of streptavidin and biotin, or one of sense and antisense nucleic acid strands that are complementary to each other), a fluorescent substance (e.g., fluorescein, fluorescein isothiocyanate, rhodamine, green fluorescent protein, and red fluorescent protein), a luminescent substance (e.g., luciferin, aequorin, acridinium ester, tris(2,2'-bipyridine)ruthenium, and luminol), a radioactive isotope (e.g., ³H, ¹⁴C, ³²P, ³⁵S, and ¹²⁵I), and a gold colloid.

According to an embodiment of the present disclosure, the drug is a small-molecule drug that can bind to the antibody or antigen-binding fragment, the recombinant protein, or the multispecific antibody.

According to an embodiment of the present disclosure, the protein tag includes, but is not limited to, His tag, Flag tag, GST tag, MBP tag, SUMO tag, and C-Myc tag.

According to an embodiment of the present disclosure, the modifier should be understood in a broad sense and may refer to a substance used to modify a protein. Exemplarily, it may be polyethylene glycol or a derivative thereof.

According to an embodiment of the present disclosure, the chemotherapeutic agent includes, but is not limited to, albumin-bound paclitaxel, cyclophosphamide, ifosfamide, melphalan, methotrexate, fluorouracil, actinomycin D, and vincristine.

It should be noted that, a method for binding the coupling moiety to the antibody or antigen-binding fragment, the recombinant protein, or the multispecific antibody can use methods known in the art, for example: the physical adsorption method, the covalent binding method, the method using affinity substances (such as biotin and streptavidin), and the ion binding method.

In yet another aspect of the present disclosure, the present disclosure provides a kit. According to an embodiment of the present disclosure, the kit includes: the above-described antibody or antigen-binding fragment, the above-described recombinant protein, the above-described multispecific antibody, the above-described nucleic acid molecule, the above-described expression vector, or the above-described recombinant cell. As can be seen from the above, the above-described antibody or antigen-binding fragment can bind to NKp46, and recombinant protein and multispecific antibody containing the above-described antibody or antigen-binding fragment can bind to NKp46. In addition, under suitable conditions, the nucleic acid molecule, the expression vector, or the recombinant cell can express the antibody or antigen-binding fragment. Further, the kit containing the above-described substances can effectively bind to NKp46 and can be used to effectively detect NKp46. The kit can be used for scientific research, such as qualitative or quantitative detection of NKp46 in biological samples, and can also be used to determine a state of an individual, such as determining whether the NKp46 level is excessively higher or lower than the normal level after obtaining the NKp46 level of the individual. The biological samples may be cells, tissues, blood, etc. Use

In yet another aspect of the present disclosure, the present disclosure provides use of the above-described antibody or antigen-binding fragment, the above-described recombinant protein, the above-described multispecific antibody, the above-described pharmaceutical composition, or the above-described conjugate in the manufacture of a medicament for preventing and/or treating an NKp46-mediated disease.

In yet another aspect of the present disclosure, the present disclosure provides use of the above-described antibody or antigen-binding fragment, the above-described recombinant protein, the above-described multispecific antibody, the above-described pharmaceutical composition, or the above-described conjugate in the prevention and/or treatment of an NKp46-mediated disease or the detection of NKp46.

In yet another aspect of the present disclosure, the present disclosure provides the above-described antibody or antigen-binding fragment, the above-described recombinant protein, the above-described multispecific antibody, the above-described pharmaceutical composition, or the above-described conjugate, for use in the prevention and/or treatment of an NKp46-mediated disease or the detection of NKp46.

According to an embodiment of the present disclosure, the above-described uses may further include at least one of the following additional technical features.

According to an embodiment of the present disclosure, the NKp46-mediated disease includes at least one selected from a tumor or cancer, a disease caused by transplant rejection, an autoimmune disease, and an infectious disease.

According to an embodiment of the present disclosure, the cancer is at least one of lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

In yet another aspect of the present disclosure, the present disclosure provides use of the above-described antibody or antigen-binding fragment, the above-described recombinant protein, the above-described multispecific antibody, the above-described conjugate, or the above-described kit in the manufacture of a product for detecting NKp46.

### Method

In yet another aspect of the present disclosure, the present disclosure provides a method for treating or preventing a cancer or tumor. According to an embodiment of the present disclosure, the method includes: administering to a subject a pharmaceutically acceptable amount of the above-described antibody or antigen-binding fragment, the above-described recombinant protein, the above-described multispecific antibody, the above-described pharmaceutical composition, or the above-described conjugate. The method of the present disclosure can effectively treat or prevent cancers or tumors.

The effective amount of the antibody or antigen-binding fragment, the recombinant protein, the multispecific antibody, the pharmaceutical composition, or the conjugate of the present disclosure can vary with the administration mode, the severity of the disease to be treated, etc. The selection of the preferred effective amount can be determined by those of ordinary skill in the art based on various factors (e.g., through clinical trials). Such factors include, but are not limited to: pharmacokinetic parameters of the active component such as bioavailability, metabolism, and half-life period, the severity of the disease to be treated in the patient, the patient's body weight, the immune status of the patient, and the route of administration. For example, depending on exigencies of the therapeutic situation, the doses can be administered several times a day separately, or the dose can be proportionally reduced.

The antibody or antigen-binding fragment, the recombinant protein, the multispecific antibody, the pharmaceutical composition, or the conjugate of the present disclosure can be incorporated into drugs suitable for parenteral administration (e.g., intravenous, subcutaneous, intraperitoneal, and intramuscular). These drugs can be prepared in various forms, such as liquid, semi-solid, and solid dosage forms, including but not limited to liquid solutions (e.g., injection solutions and infusion solutions) or freeze-dried powders. Typical drugs are in the form of injection solutions or infusion solutions. The above-described antibody or antigen-binding fragment, recombinant protein, multispecific antibody, pharmaceutical composition, or conjugate can be administered by intravenous infusion or injection, or intramuscular or subcutaneous injection.

According to an embodiment of the present disclosure, an administration route of the method is subcutaneous injection or intravenous injection.

In yet another aspect of the present disclosure, the present disclosure provides a method for detecting NKp46. According to an embodiment of the present disclosure, the method includes: contacting a sample to be detected with the above-described antibody or antigen-binding fragment, the above-described recombinant protein, the above-described multispecific antibody, the above-described conjugate, or the above-described kit to form an immune complex.

According to an embodiment of the present disclosure, whether the sample to be detected contains NKp46 is determined or a content of NKp46 is determined based on a signal of the immune complex.

According to an embodiment of the present disclosure, the immune complex further includes a second antibody, which binds to the antibody or a functional fragment thereof.

According to an embodiment of the present disclosure, the immune complex further includes the second antibody, which binds to NKp46.

A description of the sequences involved in the present disclosure is given in the following table.

| SEQ ID NO: | Sequence | Description |
|---|---|---|
| 1 | GYTFTTYW | Heavy chain CDR1 of 10D10 antibody |
| 2 | INPGNGGT | Heavy chain CDR2 of 10D10 antibody |
| 3 | AIRLRFALDY | Heavy chain CDR3 of 10D10 antibody |
| 4 | QDVRNF | Light chain CDR1 of 10D10 antibody |
| 5 | FTS | Light chain CDR2 of 10D10 antibody |
| 6 | QQGNTLPPT | Light chain CDR3 of 10D10 antibody |
| 7 | GYTFTSYW | Heavy chain CDR1 of 10E10 antibody |
| 8 | VVPGSGRT | Heavy chain CDR2 of 10E10 antibody |
| 9 | ARVGGYWDY | Heavy chain CDR3 of 10E10 antibody |
| 10 | SSVNY | Light chain CDR1 of 10E10 antibody |
| 11 | ATS | Light chain CDR2 of 10E10 antibody |
| 12 | QQWSSNPYT | Light chain CDR3 of 10E10 antibody |
| 13 | GFTFSSYA | Heavy chain CDR1 of 3B9 antibody |
| 14 | ISSGGIT | Heavy chain CDR2 of 3B9 antibody |
| 15 | VRGFLTSY | Heavy chain CDR3 of 3B9 antibody |
| 16 | QSLLNSDGKTY | Light chain CDR1 of 3B9 antibody |
| 17 | LVS | Light chain CDR2 of 3B9 antibody |
| 18 | WQGTHFWT | Light chain CDR3 of 3B9 antibody |
| 19 | QVQLVQSGAEVKKPGASVKVSCKASGYTFTTYWMHWVKQAPGQGLEWIGE INPGNGGTNYNEKFKRRATMTVDKSISTAYMELSRLRSDDTAVYYCAIRLRF ALDYWGQGTLVTVSS | Heavy chain variable region of humanized 10D10 antibody |
| 20 | DIQMTQSPSSLSASVGDRVTITCRASQDVRNFLNWFQQKPGKAPKLLIYFTSR LHSGVPSRFSGSGSGTDYTFTISSLQPEDIATYFCQQGNTLPPTFGGGTKLEIK | Light chain variable region of humanized 10D10 antibody |
| 21 | QVQLQQPGAELVRPGASVRLSCKASGYTFTTYWMHWVKLRPGQGFEWIGEI NPGNGGTNYNEKFKRKATLTVDKSSSTAYMQLSSLTSEDSAVYYCAIRLRFA LDYWGQGTSVTVSS | Heavy chain variable region of murine 10D10 antibody |
| 22 | DIQMTQTTSSLSASLGDRVTISCRASQDVRNFLNWFQQKPDGTVKLLIYFTSR LHSGVPSRFSGSGSGTDYSLTISNLEQEDIATYFCQQGNTLPPTFGGGTKLEIK | Light chain variable region of murine 10D10 antibody |
| 23 | QVQLQQSGDDLVKPGASVKLSCKASGYTFTSYWINWIKQRPGQG LEWIGRVVPGSGRTYYNEIFKGKAILTVDSYSNTAYIQLRSVSSED SAVYFCARVGGYWDYWGQGITLTVSS | Heavy chain variable region of murine 10E10 antibody |
| 24 | QIVLSQSPAILSASPGEKVTMACRASSSVNYIHWYQQRPGSSPKPW IYATSSLASGVPARFSGSGSGTSYSLTISRVEAEDAATYYCQQWSS NPYTFGGGTKLEMK | Light chain variable region of murine 10E10 antibody |
| 25 | EVKLVESGGGLVKPGGSLKLSCAPFGFTFSSYAMSWVRQTPEKRL EWVASISSGGITYYPDSVKGRFTISRDNVRNILNLQMSSLRSEDTA MYYCVRGFLTSYWGQGTLVTVSA | Heavy chain variable region of murine 3B9 antibody |
| 26 | DVVMTQTPLTLSVTIGQPASISCKSSQSLLNSDGKTYLNWLLQRPG QSPKRLISLVSKLDSGVPDRFTGSGSGTDFTLKISRVEAEDLGVYY CWQGTHFWTFGGGTKLEIK | Light chain variable region of murine 3B9 antibody |
| 27 | ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPEAAGGPSVFLFPPKPKDTLMISRTPEVTCVVVDVSHEDPEVKFN WYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHEA LHNHYTQKSLSLSPGK | Humanized heavy chain constant region (including hinge-CH2-CH3) |
| 28 | AKTTAPSVYPLAPVCGDTTGSSVTLGCLVKGYFPEPVTLTWNSGSLSSGVHTF PAVLQSDLYTLSSSVTVTSSTWPSQSITCNVAHPASSTKVDKKIEPRGPTIKPCP PCKCPAPNLLGGPSVFIFPPKIKDVLMISLSPIVTCVVVDVSEDDPDVQISWFVN NVEVHTAQTQTHREDYNSTLRVVSALPIQHQDWMSGKEFKCKVNNKDLPAPI ERTISKPKGSVRAPQVYVLPPPEEEMTKKQVTLTCMVTDFMPEDIYVEWTNN GKTELNYKNTEPVLDSDGSYFMYSKLRVEKKNWVERNSYSCSVVHEGLHNH HTTKSFSRTPGK | Murine heavy chain constant region (including hinge-CH2-CH3) |
| 29 | QVQLVQSGAEVKKPGASVKVSCKASGYTFTTYWMHWVKQAPG QGLEWIGEINPGNGGTNYNEKFKRRATMTVDKSISTAYMELSRLR SDDTAVYYCAIRLRFALDYWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCDKTH TCPPCPAPEAAGGPSVFLFPPKPKDTLMISRTPEVTCVVVDVSHED PEVKFNWYVDGVEVHNAKTKPREEQYNSTYRVVSVLTVLHQDW LNGKEYKCKVSNKALPAPIEKTISKAKGQPREPQVYTLPPSREEMT KNQVSLTCLVKGFYPSDIAVEWESNGQPENNYKTTPPVLDSDGSFF LYSKLTVDKSRWQQGNVFSCSVMHEALHNHYTQKSLSLSPGK | Heavy chain of humanized 10D10 antibody |
| 30 | DIQMTQSPSSLSASVGDRVTITCRASQDVRNFLNWFQQKPGKAPK LLIYFTSRLHSGVPSRFSGSGSGTDYTFTISSLQPEDIATYFCQQGNT LPPTFGGGTKLEIKRTVAAPSVFIFPPSDEQLKSGTASVVCLLNNFY PREAKVQWKVDNALQSGNSQESVTEQDSKDSTYSLSSTLTLSKAD YEKHKVYACEVTHQGLSSPVTKSFNRGEC | Light chain of humanized 10D10 antibody |
| 31 | QVQLQQPGAELVRPGASVRLSCKASGYTFTTYWMHWVKLRPGQ GFEWIGEINPGNGGTNYNEKFKRKATLTVDKSSSTAYMQLSSLTSE DSAVYYCAIRLRFALDYWGQGTSVTVSSASTKGPSVFPLAPSSKST SGGTAALGCLVKDYFPEPVTVSWNSGALTSGVHTFPAVLQSSGLYS LSSVVTVPSSSLGTQTYICNVNHKPSNTKVDKKVEPKSCDKTHTC PPCPAPEAAGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSREEMTKN QVSLTCLVKGFYPSDIAVEWESNGQPENNYKTTPPVLDSDGSFFLY SKLTVDKSRWQQGNVFSCSVMHEALHNHYTQKSLSLSPGK | Heavy chain of human-murine chimeric 10D10 antibody |
| 32 | DIQMTQTTSSLSASLGDRVTISCRASQDVRNFLNWFQQKPDGTVK LLIYFTSRLHSGVPSRFSGSGSGTDYSLTISNLEQEDIATYFCQQGN TLPPTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC | Light chain of human-murine chimeric 10D10 antibody |
| 33 | QVQLQQPGAELVRPGASVRLSCKASGYTFTTYWMHWVKLRPGQ GFEWIGEINPGNGGTNYNEKFKRKATLTVDKSSSTAYMQLSSLTSE DSAVYYCAIRLRFALDYWGQGTSVTVSSAKTTAPSVYPLAPVCGD TTGSSVTLGCLVKGYFPEPVTLTWNSGSLSSGVHTFPAVLQSDLYT LSSSVTVTSSTWPSQSITCNVAHPASSTKVDKKIEPRGPTIKPCPPCK CPAPNLLGGPSVFIFPPKIKDVLMISLSPIVTCVVVDVSEDDPDVQIS WFVNNVEVHTAQTQTHREDYNSTLRVVSALPIQHQDWMSGKEFK CKVNNKDLPAPIERTISKPKGSVRAPQVYVLPPPEEEMTKKQVTLT CMVTDFMPEDIYVEWTNNGKTELNYKNTEPVLDSDGSYFMYSKL RVEKKNWVERNSYSCSVVHEGLHNHHTTKSFSRTPGK | Heavy chain of murine 10D10 antibody |
| 34 | DIQMTQTTSSLSASLGDRVTISCRASQDVRNFLNWFQQKPDGTVK LLIYFTSRLHSGVPSRFSGSGSGTDYSLTISNLEQEDIATYFCQQGN TLPPTFGGGTKLEIKRADAAPTVSIFPPSSEQLTSGGASVVCFLNNF YPKDINVKWKIDGSERQNGVLNSWTDQDSKDSTYSMSSTLTLTKD EYERHNSYTCEATHKTSTSPIVKSFNRNEC | Light chain of murine 10D10 antibody |
| 35 | QVQLQQSGDDLVKPGASVKLSCKASGYTFTSYWINWIKQRPGQG LEWIGRVVPGSGRTYYNEIFKGKAILTVDSYSNTAYIQLRSVSSEDS AVYFCARVGGYWDYWGQGITLTVSSASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCDKTHTCPP CPAPEAAGGPSVFLFPPKPKDTLMISRTPEVTCVVVDVSHEDPEVK FNWYVDGVEVHNAKTKPREEQYNSTYRVVSVLTVLHQDWLNGK EYKCKVSNKALPAPIEKTISKAKGQPREPQVYTLPPSREEMTKNQV SLTCLVKGFYPSDIAVEWESNGQPENNYKTTPPVLDSDGSFFLYSK LTVDKSRWQQGNVFSCSVMHEALHNHYTQKSLSLSPGK | Heavy chain of human-murine chimeric 10E10 antibody |
| 36 | QIVLSQSPAILSASPGEKVTMACRASSSVNYIHWYQQRPGSSPKPW IYATSSLASGVPARFSGSGSGTSYSLTISRVEAEDAATYYCQQWSSN PYTFGGGTKLEMKRTVAAPSVFIFPPSDEQLKSGTASVVCLLNNFY PREAKVQWKVDNALQSGNSQESVTEQDSKDSTYSLSSTLTLSKAD YEKHKVYACEVTHQGLSSPVTKSFNRGEC | Light chain of human-murine chimeric 10E10 antibody |
| 37 | QVQLQQSGDDLVKPGASVKLSCKASGYTFTSYWINWIKQRPGQG LEWIGRVVPGSGRTYYNEIFKGKAILTVDSYSNTAYIQLRSVSSEDS AVYFCARVGGYWDYWGQGITLTVSSAKTTAPSVYPLAPVCGDTT GSSVTLGCLVKGYFPEPVTLTWNSGSLSSGVHTFPAVLQSDLYTLS SSVTVTSSTWPSQSITCNVAHPASSTKVDKKIEPRGPTIKPCPPCKCP APNLLGGPSVFIFPPKIKDVLMISLSPIVTCVVVDVSEDDPDVQISW FVNNVEVHTAQTQTHREDYNSTLRVVSALPIQHQDWMSGKEFKC KVNNKDLPAPIERTISKPKGSVRAPQVYVLPPPEEEMTKKQVTLTC MVTDFMPEDIYVEWTNNGKTELNYKNTEPVLDSDGSYFMYSKLR VEKKNWVERNSYSCSVVHEGLHNHHTTKSFSRTPGK | Heavy chain of murine 10E10 antibody |
| 38 | QIVLSQSPAILSASPGEKVTMACRASSSVNYIHWYQQRPGSSPKPW IYATSSLASGVPARFSGSGSGTSYSLTISRVEAEDAATYYCQQWSSN PYTFGGGTKLEMKRADAAPTVSIFPPSSEQLTSGGASVVCFLNNFY PKDINVKWKIDGSERQNGVLNSWTDQDSKDSTYSMSSTLTLTKDE YERHNSYTCEATHKTSTSPIVKSFNRNEC | Light chain of murine 10E10 antibody |
| 39 | EVKLVESGGGLVKPGGSLKLSCAPFGFTFSSYAMSWVRQTPEKRL EWVASISSGGITYYPDSVKGRFTISRDNVRNILNLQMSSLRSEDTA MYYCVRGFLTSYWGQGTLVTVSAASTKGPSVFPLAPSSKSTSGGT AALGCLVKDYFPEPVTVSWNSGALTSGVHTFPAVLQSSGLYSLSSV VTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAKGQPREPQVYTLPPSREEMTKNQVSL TCLVKGFYPSDIAVEWESNGQPENNYKTTPPVLDSDGSFFLYSKLT VDKSRWQQGNVFSCSVMHEALHNHYTQKSLSLSPGK | Heavy chain of human-murine chimeric 3B9 antibody |
| 40 | DVVMTQTPLTLSVTIGQPASISCKSSQSLLNSDGKTYLNWLLQRPG QSPKRLISLVSKLDSGVPDRFTGSGSGTDFTLKISRVEAEDLGVYY CWQGTHFWTFGGGTKLEIKRTVAAPSVFIFPPSDEQLKSGTASVVC LLNNFYPREAKVQWKVDNALQSGNSQESVTEQDSKDSTYSLSSTL TLSKADYEKHKVYACEVTHQGLSSPVTKSFNRGEC | Light chain of human-murine chimeric 3B9 antibody |
| 41 | EVKLVESGGGLVKPGGSLKLSCAPFGFTFSSYAMSWVRQTPEKRL EWVASISSGGITYYPDSVKGRFTISRDNVRNILNLQMSSLRSEDTA MYYCVRGFLTSYWGQGTLVTVSAAKTTAPSVYPLAPVCGDTTGS SVTLGCLVKGYFPEPVTLTWNSGSLSSGVHTFPAVLQSDLYTLSSS VTVTSSTWPSQSITCNVAHPASSTKVDKKIEPRGPTIKPCPPCKCPA PNLLGGPSVFIFPPKIKDVLMISLSPIVTCVVVDVSEDDPDVQISWF VNNVEVHTAQTQTHREDYNSTLRVVSALPIQHQDWMSGKEFKCK VNNKDLPAPIERTISKPKGSVRAPQVYVLPPPEEEMTKKQVTLTCM VTDFMPEDIYVEWTNNGKTELNYKNTEPVLDSDGSYFMYSKLRV EKKNWVERNSYSCSVVHEGLHNHHTTKSFSRTPGK | Heavy chain of murine 3B9 antibody |
| 42 | DVVMTQTPLTLSVTIGQPASISCKSSQSLLNSDGKTYLNWLLQRPG QSPKRLISLVSKLDSGVPDRFTGSGSGTDFTLKISRVEAEDLGVYY CWQGTHFWTFGGGTKLEIKRADAAPTVSIFPPSSEQLTSGGASVV CFLNNFYPKDINVKWKIDGSERQNGVLNSWTDQDSKDSTYSMSS TLTLTKDEYERHNSYTCEATHKTSTSPIVKSFNRNEC | Heavy chain of murine 3B9 antibody |
| 43 | PKSSDKTHTCPPCPAPEAAGGPSVFLFPPKPKDTLMISRTPEVTCV VVDVSHEDPEVKFNWYVDGVEVHNAKTKPREEQYNSTYRVVSV LTVLHQDWLNGKEYKCKVSNKALPAPIEKTISKAKGQPREPQVYT LPPCREEMTKNQVSLWCLVKGFYPSDIAVEWESNGQPENNYKTT PPVLDSDGSFFLYSKLTVDKSRWQQGNVFSCSVMHEALHNHYTQ KSLSLSPGK | Fc fragment of NKp46 antibody (first Fc fragment, including hinge-CH2-CH3) |
| 44 | GGGGSGGGGSGGGGS | First linking peptide |
| 45 | QVQLQQPGAELVRPGASVRLSCKASGYTFTTYWMHWVKLRPGQ GFEWIGEINPGNGGTNYNEKFKRKATLTVDKSSSTAYMQLSSLTS EDSAVYYCAIRLRFALDYWGQGTSVTVSSGGGGSGGGGSGGGGS DIQMTQTTSSLSASLGDRVTISCRASQDVRNFLNWFQQKPDGTVK LLIYFTSRLHSGVPSRFSGSGSGTDYSLTISNLEQEDIATYFCQQGN TLPPTFGGGTKLEIK | Humanized h10D10 ScFv |
| 46 | QVQLVQSGAEVKKPGASVKVSCKASGYTFTTYWMHWVKQAPG QGLEWIGEINPGNGGTNYNEKFKRRATMTVDKSISTAYMELSRLR SDDTAVYYCAIRLRFALDYWGQGTLVTVSSGGGGSGGGGSGGGG SDIQMTQSPSSLSASVGDRVTITCRASQDVRNFLNWFQQKPGKAP KLLIYFTSRLHSGVPSRFSGSGSGTDYTFTISSLQPEDIATYFCQQGN TLPPTFGGGTKLEIKGGGGSPKSSDKTHTCPPCPAPEAAGGPSVFLF PPKPKDTLMISRTPEVTCVVVDVSHEDPEVKFNWYVDGVEVHNA KTKPREEQYNSTYRVVSVLTVLHQDWLNGKEYKCKVSNKALPAP IEKTISKAKGQPREPQVYTLPPCREEMTKNQVSLWCLVKGFYPSDI AVEWESNGQPENNYKTTPPVLDSDGSFFLYSKLTVDKSRWQQGN VFSCSVMHEALHNHYTQKSLSLSPGK | Humanized h10D10 ScFv-Fc |
| 47 | QVQLQQPGAELVRPGASVRLSCKASGYTFTTYWMHWVKLRPGQ GFEWIGEINPGNGGTNYNEKFKRKATLTVDKSSSTAYMQLSSLTSE DSAVYYCAIRLRFALDYWGQGTSVTVSSGGGGSGGGGSGGGGSD IQMTQTTSSLSASLGDRVTISCRASQDVRNFLNWFQQKPDGTVKL LIYFTSRLHSGVPSRFSGSGSGTDYSLTISNLEQEDIATYFCQQGNT LPPTFGGGTKLEIK | Murine 10D10 ScFv |
| 48 | QVQLQQPGAELVRPGASVRLSCKASGYTFTTYWMHWVKLRPGQ GFEWIGEINPGNGGTNYNEKFKRKATLTVDKSSSTAYMQLSSLTSE DSAVYYCAIRLRFALDYWGQGTSVTVSSGGGGSGGGGSGGGGSD IQMTQTTSSLSASLGDRVTISCRASQDVRNFLNWFQQKPDGTVKL LIYFTSRLHSGVPSRFSGSGSGTDYSLTISNLEQEDIATYFCQQGNT LPPTFGGGTKLEIKGGGGSPKSSDKTHTCPPCPAPEAAGGPSVFLFP PKPKDTLMISRTPEVTCVVVDVSHEDPEVKFNWYVDGVEVHNAK TKPREEQYNSTYRVVSVLTVLHQDWLNGKEYKCKVSNKALPAPIE KTISKAKGQPREPQVYTLPPCREEMTKNQVSLWCLVKGFYPSDIA VEWESNGQPENNYKTTPPVLDSDGSFFLYSKLTVDKSRWQQGNV FSCSVMHEALHNHYTQKSLSLSPGK | Murine 10D10 ScFv-Fc |
| 49 | PKSCDKTHTCPPCPAPEAAGGPSVFLFPPKPKDTLMISRTPEVTCV VVDVSHEDPEVKFNWYVDGVEVHNAKTKPREEQYNSTYRVVSV LTVLHQDWLNGKEYKCKVSNKALPAPIEKTISKAKGQPREPQVCT LPPSREEMTKNQVSLSCAVKGFYPSDIAVEWESNGQPENNYKTTP PVLDSDGSFFLVSKLTVDKSRWQQGNVFSCSVMHEALHNHYTQK SLSLSPGK | Fc fragment of MICA antibody (second Fc fragment, including hinge-CH2-CH3) |
| 50 | PKSCDKTHTCPPCPAPELLGGPSVFLFPPKPKDTLMISRTPEVTCVV VDVSHEDPEVKFNWYVDGVEVHNAKTKPREEQYNSTYRVVSVL TVLHQDWLNGKEYKCKVSNKALPAPIEKTISKAKGQPREPQVYTL PPSREEMTKNQVSLTCLVKGFYPSDIAVEWESNGQPENNYKTTPP VLDSDGSFFLYSKLTVDKSRWQQGNVFSCSVMHEALHNHYTQKS LSLSPGK | Amino acid sequence of wild-type human IgG1 Fc (including hinge-CH2-CH3) |
| 51 | QVQLQESGPGLVKPSETLSLTCTVSGFSLTTYGVHWIRQPPGKGLE WIGMIWTDATTDYNAAFISRVTISVDTSKNQFSLKLSSVTAADTA VYYCARKGHGYYYAMDYWGQGTLVTVSS | MICA heavy chain variable region |
| 52 | EIVLTQSPGTLSLSPGERATLSCTASSSVSSSYLHWYQQKPGQAPR LLIYSTSNLASGIPDRFSGSGSGTDFTLTISRLEPEDFAVYYCHQYH RSPFTFGQGTKLEIK | MICA light chain variable region |
| 53 | QVQLQESGPGLVKPSETLSLTCTVSGFSLTTYGVHWIRQPPGKGLE WIGMIWTDATTDYNAAFISRVTISVDTSKNQFSLKLSSVTAADTAV YYCARKGHGYYYAMDYWGQGTLVTVSSASTKGPSVFPLAPSSKS TSGGTAALGCLVKDYFPEPVTVSWNSGALTSGVHTFPAVLQSSGLY SLSSVVTVPSSSLGTQTYICNVNHKPSNTKVDKKVEPKSCDKTHT CPPCPAPEAAGGPSVFLFPPKPKDTLMISRTPEVTCVVVDVSHEDP EVKFNWYVDGVEVHNAKTKPREEQYNSTYRVVSVLTVLHQDWL NGKEYKCKVSNKALPAPIEKTISKAKGQPREPQVCTLPPSREEMTK NOVSLSCAVKGFYPSDIAVEWESNGOPENNYKTTPPVLDSDGSFFL VSKLTVDKSRWQQGNVFSCSVMHEALHNHYTQKSLSLSPGK | MICA antibody heavy chain |
| 54 | EIVLTQSPGTLSLSPGERATLSCTASSSVSSSYLHWYQQKPGQAPRL LIYSTSNLASGIPDRFSGSGSGTDFTLTISRLEPEDFAVYYCHQYHRS PFTFGQGTKLEIKRTVAAPSVFIFPPSDEQLKSGTASVVCLLNNFYP REAKVQWKVDNALQSGNSQESVTEQDSKDSTYSLSSTLTLSKAD YEKHKVYACEVTHQGLSSPVTKSFNRGEC | MICA antibody light chain |
| 55 | | Humanized light chain CL region |
| 56 | RADAAPTVSIFPPSSEQLTSGGASVVCFLNNFYPKDINVKWKIDGS ERQNGVLNSWTDQDSKDSTYSMSSTLTLTKDEYERHNSYTCEAT HKTSTSPIVKSFNRNEC | Murine light chain CL region |
| 57 | QVQLQQSGDDLVKPGASVKLSCKASGYTFTSYWINWIKQRPGQG LEWIGRVVPGSGRTYYNEIFKGKAILTVDSYSNTAYIQLRSVSSEDS AVYFCARVGGYWDYWGQGITLTVSSGGGGSGGGGSGGGGSQIVL SQSPAILSASPGEKVTMACRASSSVNYIHWYQQRPGSSPKPWIYAT SSLASGVPARFSGSGSGTSYSLTISRVEAEDAATYYCQQWSSNPYT FGGGTKLEMK | Murine 10E10 ScFv |
| 58 | QVQLQQSGDDLVKPGASVKLSCKASGYTFTSYWINWIKQRPGQG LEWIGRVVPGSGRTYYNEIFKGKAILTVDSYSNTAYIQLRSVSSEDS AVYFCARVGGYWDYWGQGITLTVSSGGGGSGGGGSGGGGSQIVL SQSPAILSASPGEKVTMACRASSSVNYIHWYQQRPGSSPKPWIYAT SSLASGVPARFSGSGSGTSYSLTISRVEAEDAATYYCQQWSSNPYT FGGGTKLEMKGGGGSPKSSDKTHTCPPCPAPEAAGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPCREEMTKNQVSLWCLVKGFYPSDIAVEW ESNGQPENNYKTTPPVLDSDGSFFLYSKLTVDKSRWQQGNVFSCS VMHEALHNHYTQKSLSLSPGK | Murine 10E10 ScFv-Fc |
| 59 | EVKLVESGGGLVKPGGSLKLSCAPFGFTFSSYAMSWVRQTPEKRL EWVASISSGGITYYPDSVKGRFTISRDNVRNILNLQMSSLRSEDTA MYYCVRGFLTSYWGQGTLVTVSAGGGGSGGGGSGGGGSDVVMT QTPLTLSVTIGQPASISCKSSQSLLNSDGKTYLNWLLQRPGQSPKRL ISLVSKLDSGVPDRFTGSGSGTDFTLKISRVEAEDLGVYYCWQGTH FWTFGGGTKLEIK | Murine 3B9 ScFv |
| 60 | EVKLVESGGGLVKPGGSLKLSCAPFGFTFSSYAMSWVRQTPEKRL EWVASISSGGITYYPDSVKGRFTISRDNVRNILNLQMSSLRSEDTA MYYCVRGFLTSYWGQGTLVTVSAGGGGSGGGGSGGGGSDVVMT QTPLTLSVTIGQPASISCKSSQSLLNSDGKTYLNWLLQRPGQSPKRL ISLVSKLDSGVPDRFTGSGSGTDFTLKISRVEAEDLGVYYCWQGTH FWTFGGGTKLEIKGGGGSPKSSDKTHTCPPCPAPEAAGGPSVFLFP PKPKDTLMISRTPEVTCVVVDVSHEDPEVKFNWYVDGVEVHNAK TKPREEQYNSTYRVVSVLTVLHQDWLNGKEYKCKVSNKALPAPIE KTISKAKGQPREPQVYTLPPCREEMTKNQVSLWCLVKGFYPSDIA VEWESNGQPENNYKTTPPVLDSDGSFFLYSKLTVDKSRWQQGNV FSCSVMHEALHNHYTQKSLSLSPGK | Murine 3B9 ScFv-Fc |
| 61 | MSSTLPALLCVGLCLSQRISAQQQTLPKPFIWAEPHFMVPKEKQVT ICCQGNYGAVEYQLHFEGSLFAVDRPKPPERINKVKFYIPDMNSR MAGQYSCIYRVGELWSEPSNLLDLVVTEMYDTPTLSVHPGPEVIS GEKVTFYCRLDTATSMFLLLKEGRSSHVQRGYGKVQAEFPLGPVT TAHRGTYRCFGSYNNHAWSFPSEPVKLLVTGDIENTSLAPEDPTFP ADTWGTYLLTTETGLQKDHALWDHTAQNLLRMGLAFLVLVALV WFLVEDWLSRKRTRERASRASTWEGRRRLNTQTL | NKp46 protein |
| 62 | QVQLQQPGSELVRPGASVKLSCKASGYTFTSYWMHWVKQRPGQ GLEWIGTIYPGSDITNYDEKFKNKATLTVDTSSSTAYMQLSSLTSED SAVYYCTRDGRFANWGQGTLVTVSAASTKGPSVFPLAPSSKSTSG GTAALGCLVKDYFPEPVTVSWNSGALTSGVHTFPAVLQSSGLYSLS SVVTVPSSSLGTQTYICNVNHKPSNTKVDKKVEPKSCDKTHTCPP CPAPEAAGGPSVFLFPPKPKDTLMISRTPEVTCVVVDVSHEDPEVK FNWYVDGVEVHNAKTKPREEQYNSTYRVVSVLTVLHQDWLNGK EYKCKVSNKALPAPIEKTISKAKGQPREPQVYTLPPSREEMTKNQV SLTCLVKGFYPSDIAVEWESNGQPENNYKTTPPVLDSDGSFFLYSK LTVDKSRWQQGNVFSCSVMHEALHNHYTQKSLSLSPGK | IN0321 heavy chain |
| 63 | EIQMTQSPSSLSASLGERVSLTCRASQDIGSSLNWLQQEPDGTIKRL IYATSSLDSGVPKRFSGSRSGSDYSLTISSLESEDFVDYYCLQYASSP YTFGGGTKLEIKRTVAAPSVFIFPPSDEQLKSGTASVVCLLNNFYPR EAKVQWKVDNALQSGNSQESVTEQDSKDSTYSLSSTLTLSKADY EKHKVYACEVTHQGLSSPVTKSFNRGEC | IN0321 light chain |
| 64 | QVTLKESGPGILQPSQTLSLTCSFSGFSLSTYGIGVGWNRQPSGKG LEWLAHIWWNDNEYYNIDLKSRLTISKDTSNNQVFLKIASVDTAD TATYYCVRGNYRYARGYVMDYWGQGTSVTVSSASTKGPSVFPLA PSSKSTSGGTAALGCLVKDYFPEPVTVSWNSGALTSGVHTFPAVLQ SSGLYSLSSVVTVPSSSLGTQTYICNVNHKPSNTKVDKKVEPKSCD KTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRE EMTKNQVSLTCLVKGFYPSDIAVEWESNGQPENNYKTTPPVLDSD GSFFLYSKLTVDKSRWQQGNVFSCSVMHEALHNHYTQKSLSLSPG K | Jeru4602 heavy chain |
| 65 | DIVLTQSPASLAVSLGQRATISCRASESVEYYGTSLMQWYQQKPGQ PPKLLIYAASNVESGVPARFSGSGSGTDFSLNIHPVEEDDFAMYFC QQNRKVPWTFGGGTKLEIKRTVAAPSVFIFPPSDEQLKSGTASVVC LLNNFYPREAKVQWKVDNALQSGNSQESVTEQDSKDSTYSLSSTL TLSKADYEKHKVYACEVTHQGLSSPVTKSFNRGEC | Jeru4602 light chain |
| 66 | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVE | CH1 region of wild-type human IgG1 |
| 67 | | Wild-type human CL region |
| 68 | GGGGS | Second linking peptide |

The solutions of the present disclosure will be illustrated with examples. Those skilled in the art can understand that the following examples are only used to illustrate the present disclosure and should not be regarded as limitations of the scope of the present disclosure. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

Unless otherwise indicated, the practice of the present disclosure will employ conventional techniques of cell biology, molecular biology (including recombinant technology), microbiology, biochemistry, and immunology, which are within the capabilities of those skilled in the art.

In the embodiments of the present disclosure, the nucleotide sequences used to prepare the expression vectors can be obtained based on their amino acid sequences using conventional methods or conventional software (such as the online program Vectorbuilder (website: https://www.vectorbuilder.cn/tool/codon-optimization.html), GeneOptimizer online program, etc.).

### Example 1: Preparation of Antibody

Balb/c mice (9-week-old, purchased from Shanghai SLAC, weighing about 20 g) were immunized with purified recombinant NKp46 extracellular region His-tag fusion protein (NKp46-His) (purchased from Acro) as an antigen to generate murine monoclonal antibody against human NKp46.

Mice were immunized three times with purified antigen and a complete Freund's adjuvant, and immune responses were detected after exsanguination via the tail vein. Sera were screened by ELISA and flow cytometry to identify mice with anti-human NKp46 immunoglobulin. Splenocytes were acquired from mice with the highest anti-NKp46 immunoglobulin and fused with murine myeloma SP2/0 cells (ATCC NO.: CRL-1581). The fused hybridoma cells were subjected to antibody screening to obtain murine mAbs.

The candidate hybridoma cells were cultured to a total number of 10⁶ cells. The cells were collected by centrifugation at 800 rpm for 10 min, and the total RNA was extracted with a Trizol kit (Invitrogen). The total RNA was used as a template to synthesize the cDNA library (Invitrogen) by reverse transcription, and cDNA was used as a template to amplify the corresponding nucleic acid sequence of the variable regions in hybridoma cells by PCR. The primer sequences used in the PCR amplification were complementary to first framework region of the antibody variable region or signal peptide region and the constant region. In a 50 µL reaction system, 2 µL of cDNA, 5 µL of 10 × PCR buffer, 2 µL of upstream and downstream primers (5 µmol), 2 µL of dNTP, 1 µL of Taq enzyme (Takara, Ex Taq), and 38 µL of H₂O were added respectively. A pre-denaturation was performed for 5 min at 95°C, and the procedure entered the temperature cycle for PCR amplification. The reaction conditions included: denaturation at 94°C for 30 s, annealing at 58°C for 45 s, extension at 72°C for 50 s, for 32 cycles, and subsequent extension at 72°C for 7 min. After sequencing the amplification products, the heavy chain variable region sequence and light chain variable region sequence of murine mAb were obtained.

In this example, three murine monoclonal antibodies were obtained, namely, murine 10D10 antibody (also referred to as 10D10 antibody), murine 10E10 antibody (also referred to as 10E10 antibody), and murine 3B9 antibody (also referred to as 3B9 antibody). The 10D10 antibody includes HCDRs with amino acid sequences set forth in SEQ ID NO: 1 to SEQ ID NO: 3 and LCDRs with amino acid sequences set forth in SEQ ID NO: 4 to SEQ ID NO: 6, a heavy chain variable region with an amino acid sequence set forth in SEQ ID NO: 21 and a light chain variable region with an amino acid sequence set forth in SEQ ID NO: 22, and a heavy chain with an amino acid sequence set forth in SEQ ID NO: 33 and a light chain with an amino acid sequence set forth in SEQ ID NO: 34. The 10E10 antibody includes HCDRs with amino acid sequences set forth in SEQ ID NO: 7 to SEQ ID NO: 9 and LCDRs with amino acid sequences set forth in SEQ ID NO: 10 to SEQ ID NO: 12, a heavy chain variable region with an amino acid sequence set forth in SEQ ID NO: 23 and a light chain variable region with an amino acid sequence set forth in SEQ ID NO: 24, and a heavy chain with an amino acid sequence set forth in SEQ ID NO: 37 and a light chain with an amino acid sequence set forth in SEQ ID NO: 38. The 3B9 antibody includes HCDRs with amino acid sequences set forth in SEQ ID NO: 13 to SEQ ID NO: 15 and the LCDRs with amino acid sequences set forth in SEQ ID NO: 16 to SEQ ID NO: 18, a heavy chain variable region with an amino acid sequence set forth in SEQ ID NO: 25 and a light chain variable region with an amino acid sequence set forth in SEQ ID NO: 26, and a heavy chain with an amino acid sequence set forth in SEQ ID NO: 41 and a light chain with an amino acid sequence set forth in SEQ ID NO: 42.

### Example 2: ELISA Assay for Murine NKp46 Antibody Binding

The binding characteristics of the three NKp46 antibodies (10D 10 antibody, 10E10 antibody, and 3B9 antibody) obtained in Example 1 were detected using the ELISA assay. The NKp46 extracellular region His tag fusion protein (NKp46-His) was coated into a 96-well plate. The signal intensity after antibody addition was used to determine the binding characteristics of the antibody to NKp46. The specific steps were as follows:

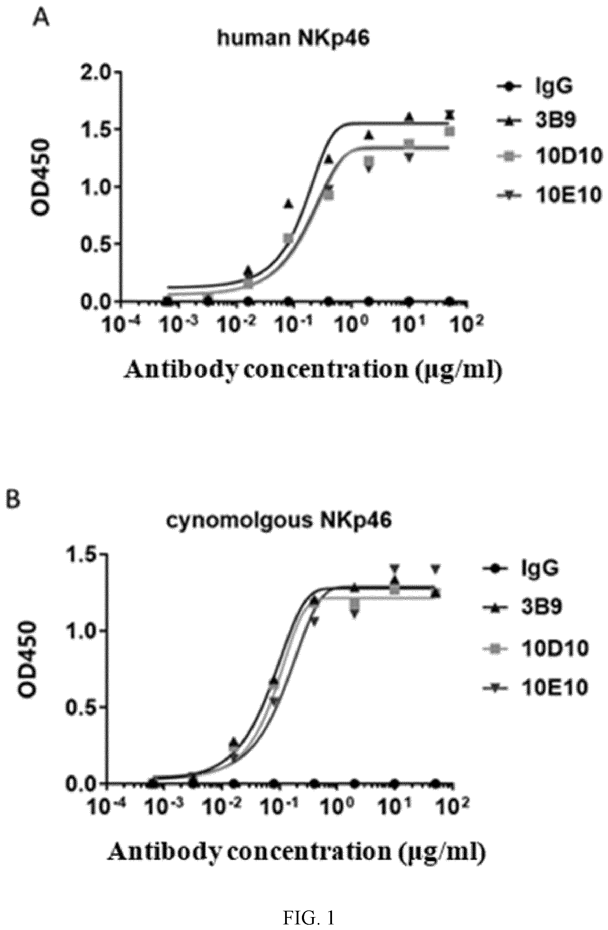

The human NKp46-His fusion protein (purchased from Acro) and cynomolgus monkey-His fusion protein (purchased from Acro) were diluted to 1 µg/mL with PBS buffer, added to a 96-well plate at 100 µL/well, and left overnight at 4°C. The PBS buffer solution was removed from the 96-well plate. The plate was washed 6 times with PBST (pH 7.2, PBS containing 0.1% Tween 20) buffer solution. PBS/10% BSA was added at 200 µL/well and incubated at 37°C for 2 hours for blocking. The blocking solution was removed. The plate was washed 6 times with PBST. NKp46 antibody to be tested diluted to an appropriate concentration with PBST/0.05% BSA was added at 100 µL/well, and incubated for 1 h at 37°C. The reaction system was removed. The plate was washed 6 times with PBST. HRP (horseradish peroxidase)-labeled anti-mouse secondary antibody diluted with PBST/0.05% BSA was added at 100 µL/well and incubated for 1 h at 37°C. The plate was washed 6 times with PBST, and TMB (tetramethylbenzidine) was added at 80 µL/well. After 3 min of incubation at room temperature, 4M sulfuric acid was added at 80 µL/well to terminate the reaction. The absorbance at 450 mm was read with a microplate reader. The results were shown in FIG. 1.

The results showed that the murine NKp46 antibodies 10D10, 10E10, and 3B9 of the present disclosure all can bind to both human and cynomolgus monkey NKp46.

### Example 3: Flow Cytometry Assay for Murine NKp46 Antibody Binding

The binding characteristics of the three NKp46 antibodies (10D10 antibody, 10E10 antibody, and 3B9 antibody) obtained in Example 1 were detected using the flow cytometry assay. Murine NKp46 antibodies were added to human peripheral blood mononuclear cells. The signal intensity after antibody addition was used to determine the binding characteristics of the antibody to PBMCs. The specific steps were as follows:

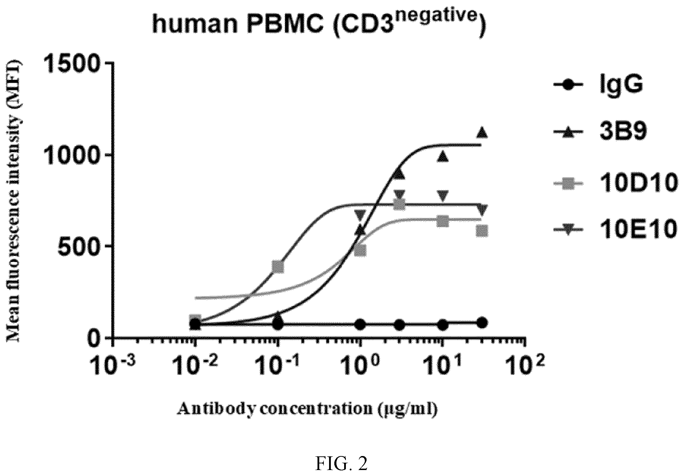

Human peripheral blood mononuclear cells (PBMCs) were diluted to 2×10⁶ cells /mL with PBS and added to a 1.5 mL EP tube at 100 µL/tube. Goat serum was added at 10 µL/tube and was blocked at 4°C for 30 min. NKp46 antibodies at different concentrations gradients were added and incubated at 4°C for 30 min. The EP tube was added with 1 mL of PBS, and centrifuged at 3500 rpm at 4°C for 5 min. The supernatant was discarded and the pellet was washed once more with PBS. After centrifugation, the supernatant was discarded, and the cells were resuspended with 100 µL/tube of PBS, and 0.1 µL/tube of Alexa-647-labeled goat anti-mouse secondary antibody (Biolegend) and 0.5 µL/tube of FITC-labeled CD3 antibody were added, and incubated at 4°C in the dark for 30 min. The pellet was washed twice with PBS and the supernatant was discarded after centrifugation. The cells were resuspended with 200 µL/tube of PBS and detected by flow cytometry. The results were shown in FIG. 2.

The results showed that the murine NKp46 antibodies 10D10, 10E10, and 3B9 of the present disclosure can bind to human PBMCs.

### Example 4: ELISA Assay for Human-Murine Chimeric NKp46 Antibody Binding

1. The inventors designed human-murine chimeric NKp46 antibodies using the heavy chain variable region and light chain variable region of the three NKp46 antibodies (10D10 antibody, 10E10 antibody, and 3B9 antibody) obtained in Example 1. The human-murine chimeric 10D10 antibody has a heavy chain with an amino acid sequence set forth in SEQ ID NO: 31 and a light chain with an amino acid sequence set forth in SEQ ID NO: 32. The human-murine chimeric 10E10 antibody has a heavy chain with an amino acid sequence set forth in SEQ ID NO: 35 and a light chain with an amino acid sequence set forth in SEQ ID NO: 36. The human-murine chimeric 3B9 antibody has a heavy chain with an amino acid sequence set forth in SEQ ID NO: 39 and a light chain with an amino acid sequence set forth in SEQ ID NO: 40. Then, human-murine chimeric 10D10 antibody (also referred to as 10D10-hIgG1LALA), human-murine chimeric 10E10 antibody (also referred to as 10E10-hIgG1LALA), and human-murine chimeric 3B9 antibody (also referred to as 3B9-hIgG1LALA) were prepared using conventional molecular biology techniques.
   The specific preparation steps were as follows: The pcDNA3.4 vectors encoding the nucleotide sequences of the heavy chain amino acid sequence and the light chain amino acid sequence of the above-described antibodies (synthesized by Nanjing GenScript) were transiently transfected into ExpiCHO-S cells (Gibco, catalog number A29127) to prepare antibodies. One day before transfection, ExpiCHO-S cells were adjusted to a density of 3×10⁶ cells/mL to 4×10⁶ cells/mL and cultured overnight with shaking at 37°C, 8% CO₂, and 95 rpm. On the day of transfection, when the cells grew to 7×10⁶ cells/mL to 1×10⁷ cells/mL with a survival rate greater than 95%, they were ready for transfection. The cells were diluted to 6×10⁶ cells/mL using freshly preheated ExpiCHO medium (Gibco, catalog number A2910002), and the above-described pcDNA3.4 plasmids carrying heavy chain and light chain (a ratio of light chain plasmid to heavy chain plasmid was 1:1) and ExpiFectamine CHO transfection reagent (Gibco, catalog number A29129) were transfected into ExpiCHO-S cells, and cultured with shaking at 37°C, 8% CO₂, and 95 rpm. 18 h to 22 h after transfection, ExpiFectamine CHO Enhancer and ExpiCHO Feed were mixed evenly and immediately added to the transfected cells, mixed evenly, and cultured with shaking at 32°C, 5% CO₂, and 95 rpm. On the 5th day after transfection, 8 mL of ExpiCHO Feed was supplemented to the cells, mixed evenly, and continued to culture. Changes in cell number and cell viability were observed daily, and the cells were harvested by centrifugation when the cell viability dropped below 80% or after 9 days of culture. The expressed supernatant was filtered with a 0.45 µm filter membrane, and the antibody with Fc domains was captured from the expressed supernatant using a protein A affinity chromatography column (purchased from Suzhou Nanomicro). After the column was equilibrated with a phosphate buffer at pH 7.2, the supernatant was passed through the affinity chromatography column and eluted with an elution buffer (100 mM citric acid, pH 2.7), and finally concentrated and replaced with a PBS buffer. The purified antibody was identified by SDS-PAGE to have a purity of more than 95%. The results showed that the above-described antibodies were finally obtained.
2. The binding characteristics of the three human-murine chimeric NKp46 antibodies (i.e., human-murine chimeric 10D10 antibody, human-murine chimeric 10E10 antibody, and human-murine chimeric 3B9 antibody) were detected using ELISA. The NKp46 extracellular region His tag fusion protein (NKp46-His) was coated into a 96-well plate. The signal intensity after antibody addition was used to determine the binding characteristics of the antibody to NKp46. The specific steps were as follows:

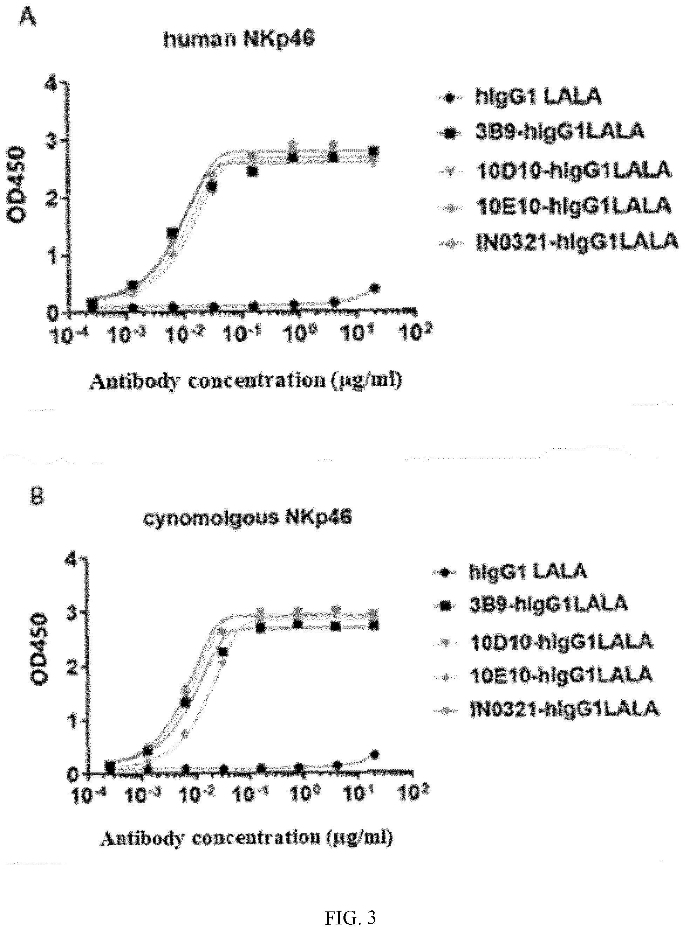

The human NKp46-His fusion protein and cynomolgus monkey-His fusion protein (for specific sources, see Example 2) were diluted to 1 µg/mL with PBS buffer, added to a 96-well plate at 100 µL/well and left overnight at 4°C. The PBS buffer solution was removed from the 96-well plate. The plate was washed 6 times with PBST (pH 7.2, PBS containing 0.1% Tween 20) buffer solution. PBS/10% BSA was added at 200 µL/well and incubated at 37°C for 2 hours for blocking. The blocking solution was removed. The plate was washed 6 times with PBST. NKp46 antibody to be tested diluted to an appropriate concentration with PBST/0.05% BSA, positive control antibody (hIgG1 LALA), and control antibody (IN0321, an amino acid sequence of the heavy chain set forth in SEQ ID NO: 62, and an amino acid sequence of the light chain set forth in SEQ ID NO: 63) were added at 100 µL/well, and incubated for 1 h at 37°C. The reaction system was removed. The plate was washed 6 times with PBST. HRP (horseradish peroxidase)-labeled anti-human secondary antibody diluted with PBST/0.05% BSA was added at 100 µL/well and incubated for 1 h at 37°C. The plate was washed 6 times with PBST, and TMB (tetramethylbenzidine) was added at 80 µL/well. After 3 min of incubation at room temperature, 4M sulfuric acid was added at 80 µL/well to terminate the reaction. The absorbance at 450 mm was read with a microplate reader. The results were shown in FIG. 3.

The results showed that the human-murine chimeric NKp46 antibodies 10D10-hIgG1LALA, 10E10-hIgG1LALA, and 3B9-hIgG1LALA of the present disclosure can bind to both human and cynomolgus monkey NKp46, and the binding abilities were comparable to that of the NKp46 antibody (IN0321, from US20190367609A1) in the Innate Pharma patent.

### Example 5: Flow Cytometry Assay for Human-Murine Chimeric NKp46 Antibody Binding

The binding characteristics of the two human-murine chimeric NKp46 antibodies (i.e., human-murine chimeric 10D10 antibody and human-murine chimeric 3B9 antibody) prepared in Example 4 were detected using the flow cytometry assay. Human-murine chimeric NKp46 antibodies were added to human and cynomolgus monkey peripheral blood mononuclear cells. The signal intensity after antibody addition was used to determine the binding characteristics of the antibody to PBMCs. The specific steps were as follows:

Human and cynomolgus monkey peripheral blood mononuclear cells (PBMCs) were diluted to 2×10⁶ cells/mL with PBS and added to 1.5 mL EP tube at 100 µL/tube. Goat serum was added at 10 µL/tube and was blocked at 4°C for 30 min. NKp46 antibodies at different concentrations gradients and the positive control antibody (hIgG1 LALA) were added and incubated at 4°C for 30min. The EP tube was added with 1 mL of PBS, and centrifuged at 3500 rpm at 4°C for 5 min. The supernatant was discarded and the pellet was washed once more with PBS. After centrifugation, the supernatant was discarded, and the cells were resuspended with 100 µL/tube of PBS, and 0.1 µL/tube of Alexa-647 labeled rat anti-human IgG-Fc secondary antibody (Biolegend) and 0.5 µL/tube BV510-labeled CD16 antibody were added, and incubated at 4°C in the dark for 30 min. The pellet was washed twice with PBS and the supernatant was discarded after centrifugation. The cells were resuspended with 200 µL/tube of PBS and detected by flow cytometry. The results were shown in FIG. 2.

The results showed that the human-murine chimeric NKp46 antibodies 10D10-hIgG1LALA and 3B9-hIgG1LALA of the present disclosure can bind to both human and cynomolgus monkey PBMCs.

### Example 6: Humanization of Mouse Antibody

With reference to the light chain variable region sequence and the heavy chain variable region sequence of the murine 10D10 antibody obtained in Example 1, the humanized template that best matched the non-CDRs was selected. The murine antibody CDRs were grafted onto the selected humanized template to replace the CDRs of the human template. Then, based on the three-dimensional structure of the murine antibody, reverse mutation were performed on the embedded residues, the residues that have direct interaction with the CDRs, and the residues that have important influence on the conformation of VL and VH to obtain a humanized antibody (also referred to as humanized 10D10 antibody or h10D10-hIgG1LALA, an amino acid sequence of the heavy chain set forth in SEQ ID NO: 29, and an amino acid sequence of the light chain set forth in SEQ ID NO: 30). The humanized 10D10 antibody was prepared using conventional molecular biology techniques (see step 1 of Example 4 for details, with the only difference being the antibody sequence).

### Example 7: ELISA Assay for Humanized NKp46 Antibody Binding

The binding characteristics of the humanized 10D10 antibody prepared in Example 6 was detected using the ELISA assay. The NKp46 extracellular region His tag fusion protein (NKp46-His) was coated into a 96-well plate. The signal intensity after antibody addition was used to determine the binding characteristics of the antibody to NKp46. The specific steps were as follows:

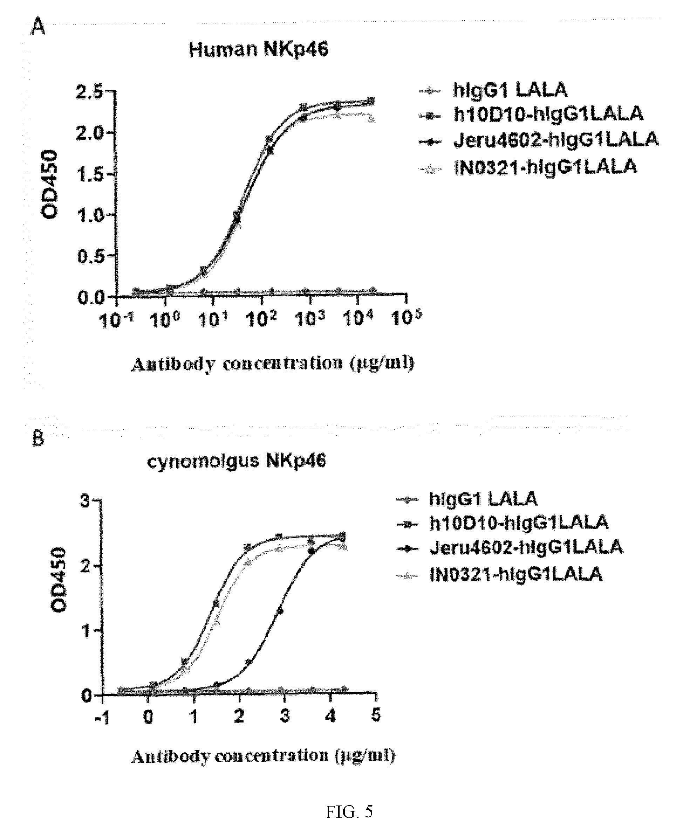

The human NKp46-His fusion protein and cynomolgus monkey-His fusion protein (for specific sources, see Example 2) were diluted to 1 µg/mL with PBS buffer, added to a 96-well plate at 100 µL/well and left overnight at 4°C. The PBS buffer solution was removed from the 96-well plate. The plate was washed 6 times with PBST (pH 7.2, PBS containing 0.1% Tween 20) buffer solution. PBS/10% BSA was added at 200 µL/well and incubated at 37°C for 2 hours for blocking. The blocking solution was removed. The plate was washed 6 times with PBST. NKp46 antibody to be tested diluted to an appropriate concentration with PBST/0.05% BSA, positive control antibody (hIgG1 LALA), control antibody 1 (IN0321, an amino acid sequence of the heavy chain set forth in SEQ ID NO:64, and an amino acid sequence of the light chain set forth in SEQ ID NO:65), and control antibody 2 (Jeru4602, an amino acid sequence of the heavy chain set forth in SEQ ID NO:62, and an amino acid sequence of the light chain set forth in SEQ ID NO:63) were added at 100 µL/well, and incubated for 1 h at 37°C. The reaction system was removed. The plate was washed 6 times with PBST. HRP (horseradish peroxidase)-labeled anti-human secondary antibody was diluted with PBST/0.05% BSA at 100 µL/well and incubated for 1 h at 37°C. The plate was washed 6 times with PBST, and TMB (tetramethylbenzidine) was added at 80 µL/well. After 3 min of incubation at room temperature, 4M sulfuric acid was added at 80 µL/well to terminate the reaction. The absorbance at 450 mm was read with a microplate reader. The results were shown in FIG. 5.

The results showed that the humanized NKp46 antibody h10D10-hIgG1LALA of the present disclosure can bind to both human and cynomolgus monkey NKp46, and the binding ability to human and monkey NKp46 was comparable to that of the NKp46 antibody in the Innate Pharma patent (IN0321, from US20190367609A1) and yet quite different from the binding ability of another NKp46 antibody Jeru4602 (from US10716864).

### Example 8: ELISA Assay for NKp46 (murine 10D10) × MICA Bispecific Antibody Binding

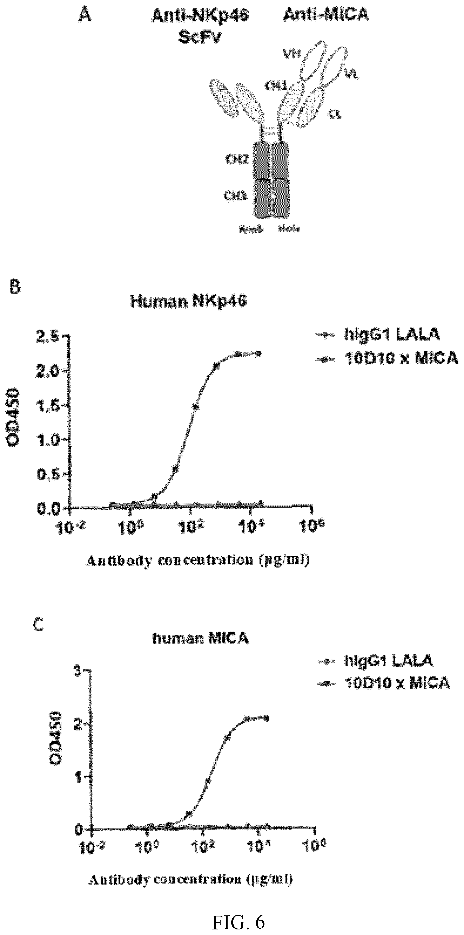

1. Murine 10D10×MICA bispecific antibody (also referred to as 10D10×MICA), murine 10E10×MICA bispecific antibody (also referred to as 10E10×MICA), and murine 3B9×MICA bispecific antibody (also referred to as 3B9×MICA) were prepared. The schematic structure of the bispecific antibodies was shown in FIG. 6A. The murine 10D10×MICA bispecific antibody includes a first peptide segment (murine 10D10 ScFv-Fc) with an amino acid sequence set forth in SEQ ID NO: 48, a second peptide segment (MICA antibody heavy chain) with an amino acid sequence set forth in SEQ ID NO: 53, and a second peptide segment (MICA antibody light chain) with an amino acid sequence set forth in SEQ ID NO: 54. The murine 10E10×MICA bispecific antibody includes a first peptide segment (murine 10E10 ScFv-Fc) with an amino acid sequence set forth in SEQ ID NO: 58, a second peptide segment (MICA antibody heavy chain) with an amino acid sequence set forth in SEQ ID NO: 53, and a second peptide segment (MICA antibody light chain) with an amino acid sequence set forth in SEQ ID NO: 54. The murine 3B9×MICA bispecific antibody includes a first peptide segment (murine 3B9 ScFv-Fc) with an amino acid sequence set forth in SEQ ID NO: 60, a second peptide segment (MICA antibody heavy chain) with an amino acid sequence set forth in SEQ ID NO: 53, and a second peptide segment (MICA antibody light chain) with an amino acid sequence set forth in SEQ ID NO: 54. This murine 10D10×MICA bispecific antibody was prepared using conventional molecular biology techniques (for details, see step 1 of Example 4, with the only difference being the antibody sequence).
2. The binding characteristics of the murine 10D10×MICA bispecific antibody prepared above were detected using the ELISA assay. The NKp46 extracellular region His tag fusion protein (NKp46-His) and the MICA extracellular region His tag fusion protein (MICA-His) were coated in a 96-well plate. The signal intensity after antibody addition was used to determine the binding characteristics of the antibody to NKp46 and MICA. The specific steps were as follows:
   The human NKp46-His fusion protein (for specific sources, see Example 2) and human MICA-His fusion protein (purchased from Acro) were diluted to 1 µg/mL with PBS buffer, added to a 96-well plate at 100 µL/well and left overnight at 4°C. The PBS buffer solution was removed from the 96-well plate. The plate was washed 6 times with PBST (pH 7.2, PBS containing 0.1% Tween 20) buffer solution. PBS/10% BSA was added at 200 µL/well and incubated at 37°C for 2 hours for blocking. The blocking solution was removed. The plate was washed 6 times with PBST. The bispecific antibody to be tested diluted to an appropriate concentration with PBST/0.05% BSA or positive control antibody (hIgG1 LALA) were added at 100 µL/well, and incubated for 1 h at 37°C. The reaction system was removed. The plate was washed 6 times with PBST. HRP (horseradish peroxidase)-labeled anti-human secondary antibody was diluted with PBST/0.05% BSA at 100 µL/well and incubated for 1h at 37°C. The plate was washed 6 times with PBST, and TMB (tetramethylbenzidine) was added at 80 µL/well. After 3 min of incubation at room temperature, 4M sulfuric acid was added at 80 µL/well to terminate the reaction. The absorbance at 450 mm was read with a microplate reader. The results were shown in FIG. 6B and FIG. 6C.

The results showed that the NKp46 (murine 10D10)×MICA bispecific antibody of the present disclosure can bind to both NKp46 and MICA.

### Example 9: ELISA Assay for NKp46 (murine 10D10) × MICA Bispecific Antibody Bridging

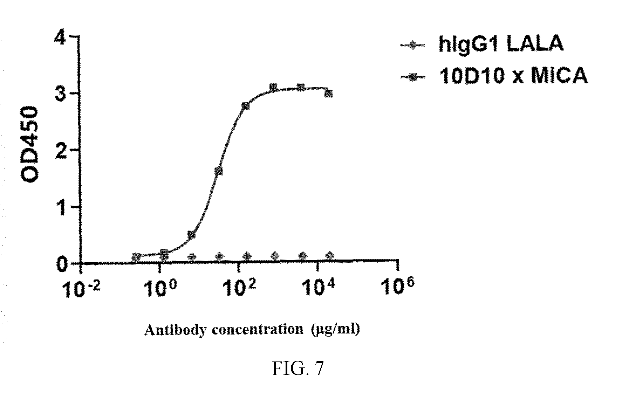

The binding characteristics of the murine 10D10×MICA bispecific antibody prepared in step 1 of Example 8 to bridge NKp46 and MICA were detected using the ELISA assay. The NKp46 extracellular region His tag fusion protein (NKp46-His) was coated into a 96-well plate. After addition of the antibody, the Biotin-labeled MICA extracellular segment α3 domain Fc fusion protein (Biotin-MICAα3-Fc) was used for detection. The signal intensity was used to determine the binding characteristics of the bispecific antibody to bridge NKp46 and MICA. The specific steps are as follows:
The human NKp46-His fusion protein (for specific sources, see Example 2) was diluted to 1 µg/mL with PBS buffer, added to a 96-well plate at 100 µL/well and left overnight at 4°C. The PBS buffer solution was removed from the 96-well plate. The plate was washed 6 times with PBST (pH 7.2, PBS containing 0.1% Tween 20) buffer solution. PBS/10% BSA was added at 200 µL/well and incubated at 37°C for 2 hours for blocking. The blocking solution was removed. The plate was washed 6 times with PBST. The bispecific antibody to be tested diluted to an appropriate concentration with PBST/0.05% BSA or positive control antibody (hIgG1 LALA) were added at 100 µL/well, and incubated for 1 h at 37°C. The reaction system was removed. The plate was washed 6 times with PBST. Biotin-MICAα3-Fc was diluted with PBST/0.05% BSA at 100 µL/well and incubated for 1h at 37°C. HRP (horseradish peroxidase)-labeled Avidin was diluted with PBST/0.05% BSA at 100 µL/well and incubated for 1h at 37°C. The plate was washed 6 times with PBST, and TMB (tetramethylbenzidine) was added at 80 µL/well. After 3 min of incubation at room temperature, 4M sulfuric acid was added at 80 µL/well to terminate the reaction. The absorbance at 450 mm was read with a microplate reader. The results were shown in FIG. 7.

The results showed that the NKp46 (murine 10D10) × MICA bispecific antibody of the present disclosure can bridge NKp46 and MICA.

### Example 10: NKp46 (murine 10D10, 10E10, and 3B9) × MICA Bispecific Antibody Promotes Killing of Tumor Cells by PBMCs

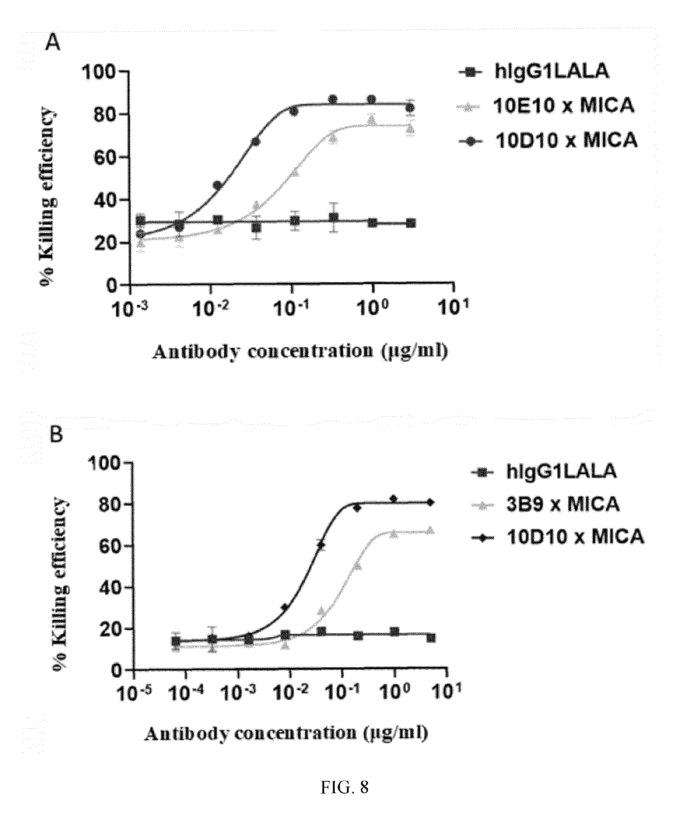

The effect of the bispecific antibody on killing A375 cells by PBMCs was detected by constructing reaction systems of tumor cells + PBMCs + bispecific antibodies of different concentrations. The specific experimental procedures were as follows:
(1) Complete RPMI-1640 medium was added to a 96-well RTCA plate at 50 µL/well followed by calibration on the machine.
(2) A375 cells were diluted to 2×10⁵ cells/mL with the complete RPMI-1640 medium and added to the RTCA plate obtained in step (1) at 50 µL/well. Then, the cell index was detected for 24 hours using the xCELLigence RTCA MP device at 37°C and 5% CO₂.
(3) NKp46×MICA bispecific antibody (murine 10D10 × MICA bispecific antibody, murine 10E10 × MICA bispecific antibody, or murine 3B9 × MICA bispecific antibody) or positive control antibody (hIgG1 LALA) was diluted into a series of concentration gradients with the complete RPMI-1640 medium and added to the RTCA plate obtained in step (2) at 20 µL/well.
(4) PBMCs (purchased from Saily Biotechnology) were diluted to 1.25×10⁶ cells/mL with the complete RPMI-1640 medium and added to the RTCA plate obtained in step (3) at 80 µL/well.
(5) The cell index of the reaction system obtained in step (4) was detected using the xCELLigence RTCA MP device for 48 hours at 37°C and 5% CO2. The results were shown in FIG. 8.

The results showed that the NKp46 (murine 10D10, 10E10, and 3B9) × MICA bispecific antibody of the present disclosure can promote the killing of tumor cells by PBMCs.

### Example 11: ELISA Assay for NKp46 (humanized 10D10) × MICA Bispecific Antibody Binding

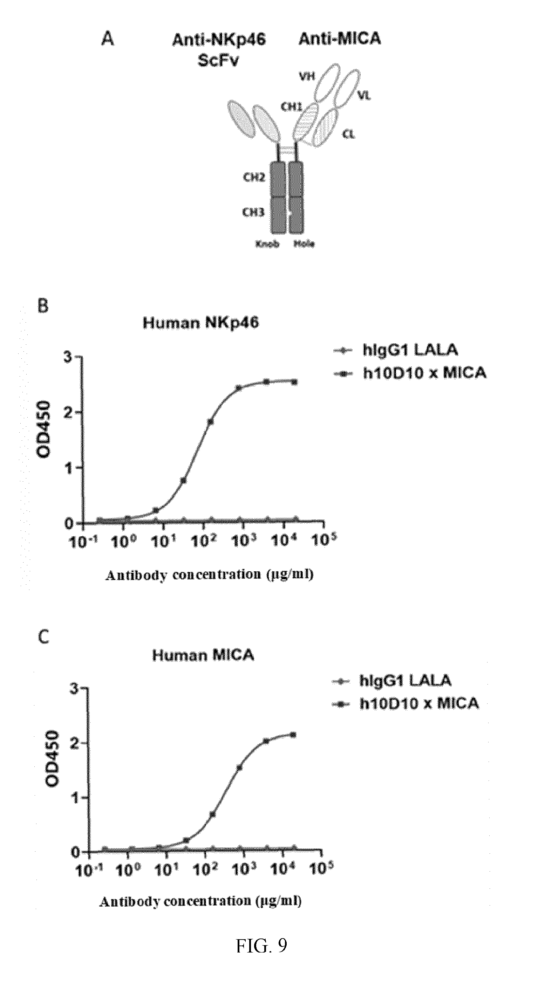

1. The humanized 10D10 × MICA bispecific antibody (also referred to as h10D10×MICA) was prepared, and the schematic structure of this bispecific antibody was shown in FIG. 9A. The humanized 10D10 (for specific sources, see Example 2) × MICA bispecific antibody includes a first peptide segment (humanized h10D10 ScFv-Fc) with an amino acid sequence set forth in SEQ ID NO: 46, a second peptide segment (MICA antibody heavy chain) with an amino acid sequence set forth in SEQ ID NO: 53, and a second peptide segment (MICA antibody light chain) with an amino acid sequence set forth in SEQ ID NO: 54. This murine 10D10×MICA bispecific antibody was prepared by conventional molecular biology techniques (for details, see step 1 of Example 4, with the only difference being the antibody sequences).
2. The binding characteristics of the humanized 10D10 × MICA bispecific antibody prepared in step 1 of this example were detected using the ELISA assay. The NKp46 extracellular region His tag fusion protein (NKp46-His) and the MICA extracellular region His tag fusion protein (MICA-His) were coated into a 96-well plate. The signal intensity after antibody addition was used to determine the binding characteristics of the antibody to NKp46 and MICA. The specific steps were as follows:
   The human NKp46-His fusion protein and human MICA-His fusion protein (purchased from Acro) were diluted to 1 µg/mL with PBS buffer, added to a 96-well plate at 100 µL/well and left overnight at 4°C. The PBS buffer solution was removed from the 96-well plate. The plate was washed 6 times with PBST (pH 7.2, PBS containing 0.1% Tween 20) buffer solution. PBS/10% BSA was added at 200 µL/well and incubated at 37°C for 2 hours for blocking. The blocking solution was removed. The plate was washed 6 times with PBST. The bispecific antibody to be tested diluted to an appropriate concentration with PBST/0.05% BSA or positive control antibody (hIgG1 LALA) were added at 100 µL/well, and incubated for 1 h at 37°C. The reaction system was removed. The plate was washed 6 times with PBST. HRP (horseradish peroxidase)-labeled anti-human secondary antibody was diluted with PBST/0.05% BSA at 100 µL/well and incubated for 1h at 37°C. The plate was washed 6 times with PBST, and TMB (tetramethylbenzidine) was added at 80 µL/well. After 3 min of incubation at room temperature, 4M sulfuric acid was added at 80 µL/well to terminate the reaction. The absorbance at 450 mm was read with a microplate reader. The results were shown in FIG. 9.

The results showed that the NKp46 (humanized 10D10) × MICA bispecific antibody of the present disclosure can bind to both NKp46 and MICA.

### Example 12: NKp46 (humanized 10D10) × MICA Bispecific Antibody Promotes Killing of Tumor Cells by PBMCs

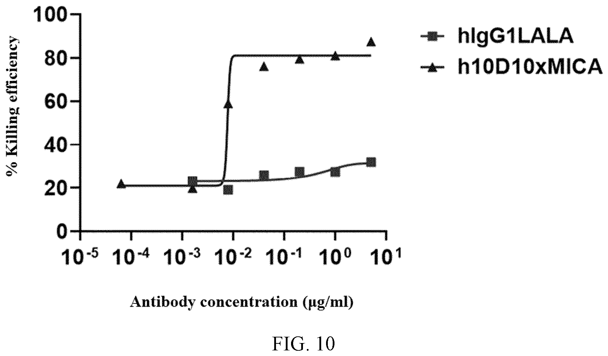

The effect of bispecific antibody on killing A375 cells by PBMCs was detected by constructing reaction systems of tumor cells + PBMC + bispecific antibodies of different concentrations. The specific experimental procedures were as follows:
(1) Complete RPMI-1640 culture medium was added to a 96-well RTCA plate at 50 µL/well, followed by calibration on the machine.
(2) A375 cells were diluted to 2×10⁵ cells/mL with the complete RPMI-1640 culture medium, and added to the RTCA plate obtained in step (1) at 50 µL/well. Then, the cell index was detected for 24 hours using the xCELLigence RTCA MP device at 37°C and 5% CO₂.
(3) NKp46×MICA bispecific antibody or positive control antibody (hIgG1 LALA) was diluted into a series of concentration gradients with the complete RPMI-1640 medium and added to the RTCA plate obtained in step (2) at 20 µL/well.
(4) PBMCs (purchased from Saily Biotechnology) were diluted to 1.25×10⁶ cells/mL with the complete RPMI-1640 medium and added to the RTCA plate obtained in step (3) at 80 µL/well.
(5) The cell index of the reaction system obtained in step (4) was detected for 48 hours using the xCELLigence RTCA MP device at 37°C and 5% CO₂. The results were shown in FIG. 10.

The results showed that the NKp46 (humanized 10D10) × MICA bispecific antibody of the present disclosure can promote the killing of tumor cells by PBMCs.

Therefore, it can be seen from the experimental results of the above-described examples that, the antibodies obtained in the present disclosure can bind to both human and cynomolgus monkey NKp46 recombinant proteins, can bind to both human and cynomolgus monkey peripheral blood mononuclear cells, and can promote anti-cancer effect of human PBMCs.

Reference throughout this specification to terms such as "an embodiment", "some embodiments", "an example", "a specific example", or "some examples" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that above embodiments are merely exemplary, and cannot be construed to limit the present disclosure. For those skilled in the art, changes, alternatives, and modifications can be made to the embodiments without departing from the scope of the present disclosure.

## Claims

1. An antibody or antigen-binding fragment, comprising a CDR selected from at least one of:
heavy chain variable region CDRs with amino acid sequences as set forth in SEQ ID NO: 1 to SEQ ID NO: 3, SEQ ID NO: 7 to SEQ ID NO: 9, or SEQ ID NO: 13 to SEQ ID NO: 15, or conservative modification forms thereof; or
light chain variable region CDRs with amino acid sequences as set forth in SEQ ID NO: 4 to SEQ ID NO: 6, SEQ ID NO: 10 to SEQ ID NO: 12, or SEQ ID NO: 16 to SEQ ID NO: 18, or conservative modification forms thereof.

2. The antibody or antigen-binding fragment according to claim 1, comprising:
a heavy chain variable region CDR1, a heavy chain variable region CDR2, and a heavy chain variable region CDR3 as set forth in SEQ ID NO: 1, SEQ ID NO: 2, and SEQ ID NO: 3, respectively; or
a heavy chain variable region CDR1, a heavy chain variable region CDR2, and a heavy chain variable region CDR3 as set forth in SEQ ID NO: 7, SEQ ID NO: 8, and SEQ ID NO: 9, respectively; or
a heavy chain variable region CDR1, a heavy chain variable region CDR2, and a heavy chain variable region CDR3 as set forth in SEQ ID NO: 13, SEQ ID NO: 14, and SEQ ID NO: 15, respectively.

3. The antibody or antigen-binding fragment according to claim 1, comprising:
a light chain variable region CDR1, a light chain variable region CDR2, and a light chain variable region CDR3 with an amino acid sequence as set forth in SEQ ID NO: 4, SEQ ID NO: 5, and SEQ ID NO: 6, respectively; or
a light chain variable region CDR1, a light chain variable region CDR2, and a light chain variable region CDR3 as set forth in set forth in SEQ ID NO: 10, SEQ ID NO: 11, and SEQ ID NO: 12, respectively; or
a light chain variable region CDR1, a light chain variable region CDR2, and a light chain variable region CDR3 as set forth in SEQ ID NO: 16, SEQ ID NO: 17, and SEQ ID NO: 18, respectively.

4. The antibody or antigen-binding fragment according to claim 1, comprising:
a heavy chain variable region CDR1, a heavy chain variable region CDR2, and a heavy chain variable region CDR3 as set forth in SEQ ID NO: 1, SEQ ID NO: 2, and SEQ ID NO: 3, respectively, and a light chain variable region CDR1, a light chain variable region CDR2, and a light chain variable region CDR3 as set forth in SEQ ID NO: 4, SEQ ID NO: 5, and SEQ ID NO: 6, respectively; or
a heavy chain variable region CDR1, a heavy chain variable region CDR2, and a heavy chain variable region CDR3 as set forth in SEQ ID NO: 7, SEQ ID NO: 8, and SEQ ID NO: 9, respectively, and a light chain variable region CDR1, a light chain variable region CDR2, and a light chain variable region CDR3 as set forth in SEQ ID NO: 10, SEQ ID NO: 11, and SEQ ID NO: 12, respectively; or
a heavy chain variable region CDR1, a heavy chain variable region CDR2, and a heavy chain variable region CDR3 as set forth in SEQ ID NO: 13, SEQ ID NO: 14, and SEQ ID NO: 15, respectively, and a light chain variable region CDR1, a light chain variable region CDR2, and a light chain variable region CDR3 as set forth in SEQ ID NO: 16, SEQ ID NO: 17, and SEQ ID NO: 18, respectively.

5. The antibody or antigen-binding fragment according to any one of claims 1 to 4, comprising a heavy chain framework region and/or a light chain framework region.

6. The antibody or antigen-binding fragment according to claim 5, wherein at least a portion of the heavy chain framework region and/or the light chain framework region is derived from at least one of a murine antibody, a human antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof, preferably at least one of a murine antibody, a human antibody, or a primate antibody.

7. The antibody or antigen-binding fragment according to any one of claims 1 to 4, comprising:
a heavy chain variable region with an amino acid sequence as set forth in any one of SEQ ID NO: 19, SEQ ID NO: 21, SEQ ID NO: 23, and SEQ ID NO: 25, or with an amino acid sequence having at least 90% homology thereto; and/or
a light chain variable region with an amino acid sequence as set forth in any one of SEQ ID NO: 20, SEQ ID NO: 22, SEQ ID NO: 24, and SEQ ID NO: 26, or with an amino acid sequence having at least 90% homology thereto.

8. The antibody or antigen-binding fragment according to any one of claims 1 to 4, comprising:
a heavy chain variable region with an amino acid sequence as set forth in SEQ ID NO: 19 or an amino acid sequence having at least 90% homology thereto, and a light chain variable region with an amino acid sequence as set forth in any one of SEQ ID NO: 20 or an amino acid sequence having at least 90% homology thereto; or
a heavy chain variable region with an amino acid sequence as set forth in SEQ ID NO: 21 or an amino acid sequence having at least 90% homology thereto, and a light chain variable region with an amino acid sequence as set forth in any one of SEQ ID NO: 22 or an amino acid sequence having at least 90% homology thereto; or
a heavy chain variable region with an amino acid sequence as set forth in SEQ ID NO: 23 or an amino acid sequence having at least 90% homology thereto, and a light chain variable region with an amino acid sequence as set forth in any one of SEQ ID NO: 24 or an amino acid sequence having at least 90% homology thereto; or
a heavy chain variable region with an amino acid sequence as set forth in SEQ ID NO: 25 or an amino acid sequence having at least 90% homology thereto, and a light chain variable region with an amino acid sequence as set forth in any one of SEQ ID NO: 26 or an amino acid sequence having at least 90% homology thereto.

9. The antibody or antigen-binding fragment according to any one of claims 1 to 4, further comprising a constant region;
wherein the constant region comprises at least one of a heavy chain constant region and a light chain constant region.

10. The antibody or antigen-binding fragment according to claim 9, wherein at least a portion of the at least one of the heavy chain constant region and the light chain constant region is derived from at least one of a murine antibody, a human antibody, a primate antibody, a bovine antibody, an equine antibody, a dairy cow antibody, a porcine antibody, an ovine antibody, a caprine antibody, a canine antibody, a feline antibody, a rabbit antibody, a camelid antibody, a donkey antibody, a deer antibody, a mink antibody, a chicken antibody, a duck antibody, a goose antibody, a turkey antibody, a gamecock antibody, or a mutant thereof.

11. The antibody or antigen-binding fragment according to claim 9, wherein:
the heavy chain constant region comprises a heavy chain constant region selected from IgG1, IgG2, IgG3, IgG4, IgA, IgM, IgE, or IgD; or
the light chain constant region comprises a light chain constant region selected from a κ-type light chain constant region or a λ-type light chain constant region.

12. The antibody or antigen-binding fragment according to claim 9, wherein the light chain constant region and the heavy chain constant region are both derived from a murine antibody or a mutant thereof, or a human antibody or a mutant thereof.

13. The antibody or antigen-binding fragment according to claim 9, wherein:
the N-terminus of the heavy chain constant region is linked to the C-terminus of the heavy chain variable region; and/or
the N-terminus of the light chain constant region is linked to the C-terminus of the light chain variable region.

14. The antibody or antigen-binding fragment according to claim 9, wherein:
the heavy chain constant region comprises a heavy chain constant region with an amino acid sequence as set forth in SEQ ID NO: 27 or an amino acid sequence having at least 80% identity thereto, or a heavy chain constant region with an amino acid sequence as set forth in SEQ ID NO: 28 or an amino acid sequence having at least 80% identity thereto; and/or
the light chain constant region comprises or is a light chain constant region with an amino acid sequence as set forth in SEQ ID NO: 55 or an amino acid sequence having at least 80% identity thereto, or the light chain constant region comprises or is a light chain constant region with an amino acid sequence as set forth in SEQ ID NO: 56 or an amino acid sequence having at least 80% identity thereto.

15. The antibody or antigen-binding fragment according to any one of claims 1 to 4, comprising:
a heavy chain with an amino acid sequence as set forth in any one of SEQ ID NO: 29, SEQ ID NO: 31, SEQ ID NO: 33, SEQ ID NO: 35, SEQ ID NO: 37, SEQ ID NO: 39, and SEQ ID NO: 41, or with an amino acid sequence having at least 90% homology thereto; and/or
a light chain with an amino acid sequence as set forth in any one of SEQ ID NO: 30, SEQ ID NO: 32, SEQ ID NO: 34, SEQ ID NO: 36, SEQ ID NO: 38, SEQ ID NO: 40, SEQ ID NO: 42, or with an amino acid sequence having at least 90% homology thereto.

16. The antibody or antigen-binding fragment according to any one of claims 1 to 4, comprising:
a heavy chain with an amino acid sequence as set forth in SEQ ID NO: 29 or an amino acid sequence having at least 80% homology thereto, and a light chain with an amino acid sequence as set forth in SEQ ID NO: 30 or an amino acid sequence having at least 80% homology thereto; or
a heavy chain with an amino acid sequence as set forth in SEQ ID NO: 31 or an amino acid sequence having at least 80% homology thereto, and a light chain with an amino acid sequence as set forth in SEQ ID NO: 32 or an amino acid sequence having at least 80% homology thereto; or
a heavy chain with an amino acid sequence as set forth in SEQ ID NO: 33 or an amino acid sequence having at least 80% homology thereto, and a light chain with an amino acid sequence as set forth in SEQ ID NO: 34 or an amino acid sequence having at least 80% homology thereto; or
a heavy chain with an amino acid sequence as set forth in SEQ ID NO: 35 or an amino acid sequence having at least 80% homology thereto, and a light chain with an amino acid sequence as set forth in SEQ ID NO: 36 or an amino acid sequence having at least 80% homology thereto; or
a heavy chain with an amino acid sequence as set forth in SEQ ID NO: 37 or an amino acid sequence having at least 80% homology thereto, and a light chain with an amino acid sequence as set forth in SEQ ID NO: 38 or an amino acid sequence having at least 80% homology thereto; or
a heavy chain with an amino acid sequence as set forth in SEQ ID NO: 39 or an amino acid sequence having at least 80% homology thereto, and a light chain with an amino acid sequence as set forth in SEQ ID NO: 40 or an amino acid sequence having at least 80% homology thereto; or
a heavy chain with an amino acid sequence as set forth in SEQ ID NO: 41 or an amino acid sequence having at least 80% homology thereto, and a light chain with an amino acid sequence as set forth in SEQ ID NO: 42 or an amino acid sequence having at least 80% homology thereto;
optionally, the antibody comprises at least one selected from a polyclonal antibody, a full-length monoclonal antibody, a Fab antibody, a Fab' antibody, a F(ab')₂ antibody, a Fv antibody, a single-chain antibody, a single-domain antibody, and a minimum recognition unit; or the antigen-binding fragment comprises at least one selected from a F(ab')₂ fragment, a Fab' fragment, a Fab fragment, a F(ab)₂ fragment, a Fv fragment, a scFv fragment, a scFv-Fc fusion protein, a scFv-Fv fusion protein, and a minimum recognition unit.

17. A recombinant protein, comprising:
the antibody or antigen-binding fragment according to any one of claims 1 to 16; and
optionally, at least one of a biologically active protein or fragment thereof, or a biologically active polypeptide or fragment thereof.

18. The recombinant protein according to claim 17, wherein the biologically active protein or the fragment thereof comprises at least one selected from a protein tag, a protein toxin or fragment thereof, a tumor necrosis factor or fragment thereof, an interferon or fragment thereof, a biological response modifier or fragment thereof, or an Fc fragment.

19. A multispecific antibody, comprising:
a first binding region, comprising the antibody or antigen-binding fragment according to any one of claims 1 to 16; and
a second binding region, having a binding activity to a first molecule.

20. The multispecific antibody according to claim 19, wherein the multispecific antibody comprises at least one selected from a bispecific antibody, a trispecific antibody, or a tetraspecific antibody, preferably a bispecific antibody.

21. The multispecific antibody according to claim 19, wherein the first molecule comprises at least one selected from a tumor-associated antigen, a virus, a bacterium, an endotoxin, a cytokine, or a cytokine receptor, preferably a tumor antigen.

22. The multispecific antibody according to claim 19, wherein the first molecule comprises at least one selected from B7H6, MICA, MICB, or PDL1, preferably MICA.

23. The multispecific antibody according to any one of claims 19 to 22, wherein the first binding region comprises a first heavy chain variable region and a first light chain variable region, wherein:
the C-terminus of the first heavy chain variable region is linked to the N-terminus of the first light chain variable region; or
the N-terminus of the first heavy chain variable region is linked to the C-terminus of the first light chain variable region.

24. The multispecific antibody according to claim 23, wherein the first binding region further comprises a first linking peptide, wherein:
the C-terminus of the first heavy chain variable region is linked to the N-terminus of the first linking peptide, and the C-terminus of the first linking peptide is linked to the N-terminus of the first light chain variable region; or
the C-terminus of the first light chain variable region is linked to the N-terminus of the first linking peptide, and the C-terminus of the first linking peptide is linked to the N-terminus of the first heavy chain variable region.

25. The multispecific antibody according to claim 23, wherein:
the first heavy chain variable region is a heavy chain variable region as defined for the antibody or antigen-binding fragment according to any one of claims 1 to 16; and/or
the first light chain variable region is a light chain variable region as defined for the antibody or antigen-binding fragment according to any one of claims 1 to 16.

26. The multispecific antibody according to claim 24, wherein the first binding region further comprises a first Fc fragment, wherein:
the C-terminus of the first heavy chain variable region is linked to the N-terminus of the first linking peptide, the C-terminus of the first linking peptide is linked to the N-terminus of the first light chain variable region, and the C-terminus of the first light chain variable region is linked to an N-terminus of the first Fc fragment; or
the C-terminus of the first light chain variable region is linked to the N-terminus of the first linking peptide, the C-terminus of the first linking peptide is linked to the N-terminus of the first heavy chain variable region, and the C-terminus of the first heavy chain variable region is linked to the N-terminus of the first Fc fragment.

27. The multispecific antibody according to claim 26, wherein, compared with an amino acid sequence of an Fc fragment from a wild-type human IgG1, the first Fc fragment has at least one mutation site selected from C220S, L234A, L235A, T366W, and S354C.

28. The multispecific antibody according to claim 27, wherein the first Fc fragment has an amino acid sequence as set forth in SEQ ID NO: 43.

29. The multispecific antibody according to claim 26, wherein the first binding region further comprises a second linking peptide;
optionally, the N-terminus of the second linking peptide is linked to the C-terminus of the first light chain variable region, and the C-terminus of the second linking peptide is linked to the N-terminus of the first Fc fragment; or
the N-terminus of the second linking peptide is linked to the C-terminus of the first heavy chain variable region, and the C-terminus of the second linking peptide is linked to the N-terminus of the first Fc fragment.

30. The multispecific antibody according to claim 29, wherein the amino acid sequence of the first linking peptide and/or the second linking peptide is (GGGGS)ₙ, where n is an integer greater than or equal to 1, preferably 1, 2, 3, 4, 5, 6, 7, 8, 9, or 10.

31. The multispecific antibody according to claim 30, wherein:
the first linking peptide has an amino acid sequence as set forth in SEQ ID NO: 44; and/or
the second linking peptide is GGGGS.

32. The multispecific antibody according to claim 19, wherein the first binding region comprises an amino acid sequence as set forth in any one of SEQ ID NO: 45, SEQ ID NO: 47, SEQ ID NO: 57, and SEQ ID NO: 59.

33. The multispecific antibody according to claim 19, wherein the first binding region has an amino acid sequence as set forth in any one of SEQ ID NO: 46, SEQ ID NO: 48, SEQ ID NO: 58, and SEQ ID NO: 60.

34. The multispecific antibody according to claim 19, wherein the second binding region comprises a first polypeptide and a second polypeptide, the first polypeptide and the second polypeptide being linked via an interchain disulfide bond; wherein:
the first polypeptide comprises a second heavy chain variable region, a CH1 region, and a second Fc fragment, wherein the C-terminus of the second heavy chain variable region is linked to the N-terminus of the CH1 region, and the C-terminus of the CH1 region is linked to the N-terminus of the second Fc fragment; and
the second polypeptide comprises a second light chain variable region and a CL region, wherein the C-terminus of the second light chain variable region is linked to the N-terminus of the CL region.

35. The multispecific antibody according to claim 34, wherein:
the CH1 region comprises a CH1 region from a wild-type human IgG1, a wild-type primate IgG1, or a wild-type murine IgG1; and/or
the second Fc fragment, compared with an amino acid sequence of an Fc fragment from a wild-type human IgG1, has at least one mutation site selected from L234A, L235A, Y349C, T366S, L368A, and Y407V.

36. The multispecific antibody according to claim 34, wherein the second Fc fragment has an amino acid sequence as set forth in SEQ ID NO: 49.

37. The multispecific antibody according to claim 34, wherein the CL region is a wild-type human CL region, a wild-type primate CL region, or a wild-type murine CL region, preferably the wild-type human CL region.

38. The multispecific antibody according to claim 34, wherein the second heavy chain variable region has an amino acid sequence as set forth in SEQ ID NO: 51.

39. The multispecific antibody according to claim 34, wherein the second light chain variable region has an amino acid sequence as set forth in SEQ ID NO: 52.

40. The multispecific antibody according to claim 34, wherein the first polypeptide has an amino acid sequence as set forth in SEQ ID NO: 53.

41. The multispecific antibody according to claim 34, wherein the second polypeptide has an amino acid sequence as set forth in SEQ ID NO: 54.

42. The multispecific antibody according to claim 19, wherein the first binding region and the second binding region are linked via a knob-into-hole structure.

43. A nucleic acid molecule, encoding the antibody or antigen-binding fragment according to any one of claims 1 to 16, the recombinant protein according to any one of claims 17 to 18, or the multispecific antibody according to any one of claims 19 to 42.

44. An expression vector, carrying the nucleic acid molecule according to claim 43.

45. A recombinant cell,
carrying the nucleic acid molecule according to claim 43 or the expression vector according to claim 44; or
expressing the antibody or antigen-binding fragment according to any one of claims 1 to 16, the recombinant protein according to any one of claims 17 to 18, or the multispecific antibody according to any one of claims 19 to 42.

46. A pharmaceutical composition, comprising:
the antibody or antigen-binding fragment according to any one of claims 1 to 16, the recombinant protein according to any one of claims 17 to 18, the multispecific antibody according to any one of claims 19 to 42, the nucleic acid molecule according to claim 43, the expression vector according to claim 44, or the recombinant cell according to claim 45; and
optionally, a pharmaceutically acceptable excipient.

47. A conjugate, comprising:
the antibody or antigen-binding fragment according to any one of claims 1 to 16, the recombinant protein according to any one of claims 17 to 18, or the multispecific antibody according to any one of claims 19 to 42; and
a coupling moiety, linked to the antibody or antigen-binding fragment, the recombinant protein, or the multispecific antibody.

48. The conjugate according to claim 47, wherein the coupling moiety comprises at least one selected from a carrier, a drug, a toxin, a cytokine, a protein tag, a modifier, and a chemotherapeutic agent.

49. A kit, comprising:
the antibody or antigen-binding fragment according to any one of claims 1 to 16, the recombinant protein according to any one of claims 17 to 18, the multispecific antibody according to any one of claims 19 to 42, the nucleic acid molecule according to claim 43, the expression vector according to claim 44, or the recombinant cell according to claim 45.

50. Use of the antibody or antigen-binding fragment according to any one of claims 1 to 16, the recombinant protein according to any one of claims 17 to 18, the multispecific antibody according to any one of claims 19 to 42, the pharmaceutical composition according to claim 46, or the conjugate according to any one of claims 47 to 48 in the manufacture of a medicament for preventing and/or treating an NKp46-mediated disease.

51. Use of the antibody or antigen-binding fragment according to any one of claims 1 to 16, the recombinant protein according to any one of claims 17 to 18, the multispecific antibody according to any one of claims 19 to 42, the pharmaceutical composition according to claim 46, or the conjugate according to any one of claims 47 to 48 in the prevention and/or treatment of an NKp46-mediated disease or the detection of NKp46.

52. The antibody or antigen-binding fragment according to any one of claims 1 to 16, the recombinant protein according to any one of claims 17 to 18, the multispecific antibody according to any one of claims 19 to 42, the pharmaceutical composition according to claim 46, or the conjugate according to any one of claims 47 to 48, for use in the prevention and/or treatment of an NKp46-mediated disease or in the detection of NKp46.

53. The use according to any one of claims 50 to 52, wherein the NKp46-mediated disease comprises at least one selected from a tumor or cancer, a disease caused by transplant rejection, an autoimmune disease, and an infectious disease.

54. The use according to claim 53, wherein the cancer is at least one of lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head and neck cancer.

55. A method for detecting NKp46, the method comprising:
contacting a sample to be detected with the antibody or antigen-binding fragment according to any one of claims 1 to 16, the recombinant protein according to any one of claims 17 to 18, the multispecific antibody according to any one of claims 19 to 42, the conjugate according to any one of claims 47 to 48, or the kit according to claim 49 to form an immune complex; and
determining whether the sample to be detected contains the NKp46 or determining a content of the NKp46 based on a signal of the immune complex.

56. A method for treating or preventing a cancer or tumor, the method comprising:
administering to a subject a pharmaceutically acceptable amount of the antibody or antigen-binding fragment according to any one of claims 1 to 16, the recombinant protein according to any one of claims 17 to 18, the multispecific antibody according to any one of claims 19 to 42, the pharmaceutical composition according to claim 46, or the conjugate according to any one of claims 47 to 48.
